# EUROPEAN PATENT SPECIFICATION

(11) **EP 1 254 240 B1**
(45) Date of publication and mention of the grant of the patent: **28.02.2007**
(21) Application number: 01908667.7
(22) Date of filing: 22.01.2001
(51) Int. Cl.: C12N 15/55, C12N 15/56, C12N 15/57, C12N 9/56, C12N 9/54, C12N 9/64, C12N 9/20, C12N 9/42, C12N 9/24, C12N 1/21, C11D 3/386, A23K 1/165, A61K 38/46, A61K 38/47, A61K 38/48, C12Q 1/34, C12Q 1/37, G01N 33/68

(54) **Hybrid protease with reduced antigenicity and its use**
Hybridprotease mit verringerter Antigenizität und deren Verwendung
PROTEASE HYBRIDE PRODUISANT UNE REPONSE IMMUNOGENE REDUITE ET PROCEDES D'UTILISATION DE CETTE PROTEIN.

(30) Priority: 08.02.2000 US 500135
(43) Date of publication of application: 06.11.2002
(73) Proprietor: GENENCOR INTERNATIONAL, INC., Palo Alto, California 94304 (US)
(72) Inventor: ESTELL, David, A., San Mateo, CA 94403 (US); HARDING, Fiona, A., Santa Clara, CA 95050 (US)
(74) Representative: Kiddle, Simon John
(86) International application number: PCT/US2001/002204
(87) International publication number: WO 2001/059130

(56) References cited:
- EP-A- 0 699 755
- WO-A-92/10755
- WO-A-98/52976
- WO-A-99/53038
- WO-A-99/61637
- GRAZIANO R F ET AL: "ENHANCING THE IMMUNOGENICITY OF A PERMISSIVE BINDING T CELL EPITOPE DERIVED FROM THE SIMIAN IMMUNODEFICIENCY VIRUS-ENCODED NEGATIVE REGULATORY FACTOR" JOURNAL OF IMMUNOLOGY, vol. 149, no. 2, 1992, pages 556-561, XP002178257 ISSN: 0022-1767
- DATABASE BIOSIS [Online] BIOSCIENCES INFORMATION SERVICE, PHILADELPHIA, PA, US; 1995 LIPFORD G B ET AL: "Peptide engineering allows cytotoxic T-cell vaccination against human papilloma virus tumour antigen, E6." Database accession no. PREV199598171988 XP002178258 & IMMUNOLOGY, vol. 84, no. 2, 1995, pages 298-303, ISSN: 0019-2805

### SEQUENCE LISTING

<110> Estell, David
   Harding, Fiona
<120> PROTEINS PRODUCING AN ALTERED IMMUNOGENIC RESPONSE AND METHODS OF MAKING AND USING THE SAME
<130> A-68893/DJB/DAV
<140> 09/500,135
   <141> 2000-02-08
<150> 09/060,872
   <151> 1998-04-15
<160> 236
<170> PatentIn Ver. 2.1
<210> 1
   <211> 1495
   <212> DNA
   <213> Bacillus amyloliquefaciens
<220>
   <221> mat_peptide
   <222> (417)..(1495)
<220>
   <221> CDS
   <222> (96)..(1244)
<220>
   <221> unsure
   <222> (96)..(98)
   <223> The nnn at positions 96 through 98 represents gtg, which is to code for methionine.
<220>
   <221> unsure
   <222> (582)..(584)
   <223> The nnn at positions 582 through 584 represents Xaa, which in a preferred embodiment (aat) is to code for asparagine, but which may also code for proline.
<220>
   <221> unsure
   <222> (585)..(587)
   <223> The nnn at positions 585 through 587 represents Xaa, which in a preferred embodiment (cct) is to code for proline, but which may also code for asparagine.
<220>
   <221> unsure
   <222> (597)..(599)
   <223> The nnn at positions 597 to 599 represents Xaa, which in a preferred embodiment (aac) is to code for asparagine, but which may also code for aspartic acid.
<220>
   <221> unsure
   <222> (678)..(680)
   <223> The nnn at positions 678 through 680 represents Xaa, which in a preferred embodiment (gca) is to code for alanine, but which may also code for serine.
<220>
   <221> unsure
   <222> (681)..(683)
   <223> The nnn at positions 681 through 683 represents Xaa, which in a preferred embodiment (tca) is to code for serine; but which may also code for alanine.
<220>
   <221> unsure
   <222> (708)..(710)
   <223> The nnn at positions 708 through 710 represents Xaa, which in a preferred embodiment (get) is to code for alanine, but which may also code for aspartic acid.
<220>
   <221> unsure
   <222> (711)..(713)
   <223> The nnn at positions 711 through 713 represents Xaa, which in a preferred embodiment (gac) is to code for aspartic acid, but which may also code for alanine.
<220>
   <221> unsure
   <222> (888)..(890)
   <223> The nnn at positions 888 through 890 represents Xaa, which in a preferred embodiment (act) is to code for threonine, but which may also code for serine.
<220>
   <221> unsure
   <222> (891)..(893)
   <223> The nnn at positions 891 through 893 represents Xaa, which in a preferred embodiment (tcc) is to code for serine, but which may also code for threonine.
<220>
   <221> unsure
   <222> (1167)..(1169)
   <223> The nnn at positions 1167 through 1169 represents Xaa, which in a preferred embodiment (gaa) is to code for glutamic acid, but which may also code for glutamine.
<400> 1
   ggtctactaa aatattattc catactatac aattaataca cagaataatc tgtctattgg 60
   ttattctgca aatgaaaaaa aggagaggat aaaga nnn aga ggc aaa aaa gta   113
                                          Xaa Arg Gly Lys Lys Val
                                                   -105
   tgg atc agt ttg ctg ttt gct tta gcg tta atc ttt acg atg gcg ttc   161
   Trp Ile Ser Leu Leu Phe Ala Leu Ala Leu Ile Phe Thr Met Ala Phe
       -100                -95                 -90
   ggc agc aca tcc tct gcc cag gcg gca ggg aaa tca aac ggg gaa aag   209
   Gly Ser Thr Ser Ser Ala Gln Ala Ala Gly Lys Ser Asn Gly Glu Lys
   -85                 -80                 -75                 -70
   aaa tat att gtc ggg ttt aaa cag aca atg agc acg atg agc gcc gct   257
   Lys Tyr Ile Val Gly Phe Lys Gln Thr Met Ser Thr Met Ser Ala Ala
                   -65                 -60                 -55
   aag aag aaa gat gtc att tct gaa aaa ggc ggg aaa gtg caa aag caa   305
   Lys Lys Lys Asp Val Ile Ser Glu Lys Gly Gly Lys Val Gln Lys Gln
               -50                 -45                 -40
   ttc aaa tat gta gac gca gct tca gct aca tta aac gaa aaa gct gta   353
   Phe Lys Tyr Val Asp Ala Ala Ser Ala Thr Leu Asn Glu Lys Ala Val
           -35                 -30                 -25
   aaa gaa ttg aaa aaa gac ccg agc gtc gct tac gtt gaa gaa gat cac   401
   Lys Glu Leu Lys Lys Asp Pro Ser Val Ala Tyr Val Glu Glu Asp His
       -20                 -15                 -10
   gta gca cat gcg tac gcg cag tcc gtg cct tac ggc gta tca caa att   449
   Val Ala His Ala Tyr Ala Gln Ser Val Pro Tyr Gly Val Ser Gln Ile
   -5                  -1   1               5                  10
   aaa gcc cct gct ctg cac tct caa ggc tac act gga tca aat gtt aaa   497
   Lys Ala Pro Ala Leu His Ser Gln Gly Tyr Thr Gly Ser Asn Val Lys
               15                  20                  25
   gta gcg gtt atc gac agc ggt atc gat tct tct cat cct gat tta aag   545
   Val Ala Val Ile Asp Ser Gly Ile Asp Ser Ser His Pro Asp Leu Lys
           30                  35                  40
   gta gca ggc gga gcc agc atg gtt cct tct gaa aca nnn nnn ttc caa   593
   Val Ala Gly Gly Ala Ser Met Val Pro Ser Glu Thr Xaa Xaa Phe Gln
       45                  50                  55
   gac nnn aac tct cac gga act cac gtt gcc ggc aca gtt gcg gct ctt   641
   Asp Xaa Asn Ser His Gly Thr His Val Ala Gly Thr Val Ala Ala Leu
   60                  65                  70                  75
   aat aac tca atc ggt gta tta ggc gtt gcg cca agc nnn nnn ctt tac   689
   Asn Asn Ser Ile Gly Val Leu Gly Val Ala Pro Ser Xaa Xaa Leu Tyr
                   80                  85                  90
   gct gta aaa gtt ctc ggt nnn nnn ggt tcc ggc caa tac agc tgg atc    737
   Ala Val Lys Val Leu Gly Xaa Xaa Gly Ser Gly Gln Tyr Ser Trp Ile
               95                  100                 105
   att aac gga atc gag tgg gcg atc gca aac aat atg gac gtt att aac    785
   Ile Asn Gly Ile Glu Trp Ala Ile Ala Asn Asn Met Asp Val Ile Asn
           110                 115                 120
   atg agc ctc ggc gga cct tct ggt tct gct gct tta aaa gcg gca gtt    833
   Met Ser Leu Gly Gly Pro Ser Gly Ser Ala Ala Leu Lys Ala Ala Val
       125                 130                 135
   gat aaa gcc gtt gca tcc ggc gtc gta gtc gtt gcg gca gcc ggt aac    881
   Asp Lys Ala Val Ala Ser Gly Val Val Val Val Ala Ala Ala Gly Asn
   140                 145                 150                 155
   gaa ggc nnn nnn ggc agc tca agc aca gtg ggc tac cct ggt aaa tac    929
   Glu Gly Xaa Xaa Gly Ser Ser Ser Thr Val Gly Tyr Pro Gly Lys Tyr
                   160                 165                 170
   cct tct gtc att gca gta ggc gct gtt gac agc agc aac caa aga gca    977
   Pro Ser Val Ile Ala Val Gly Ala Val Asp Ser Ser Asn Gln Arg Ala
               175                 180                 185
   tct ttc tca agc gta gga cct gag ctt gat gtc atg gca cct ggc gta    1025
   Ser Phe Ser Ser Val Gly Pro Glu Leu Asp Val Met Ala Pro Gly Val
           190                 195                 200
   tct atc caa agc acg ctt cct gga aac aaa tac ggg gcg tac aac ggt    1073
   Ser Ile Gln Ser Thr Leu Pro Gly Asn Lys Tyr Gly Ala Tyr Asn Gly
       205                 210                 215
   acg tca atg gca tct ccg cac gtt gcc gga gcg gct gct ttg att ctt    1121
   Thr Ser Met Ala Ser Pro His Val Ala Gly Ala Ala Ala Leu Ile Leu
   220                 225                 230                 235
   tct aag cac ccg aac tgg aca aac act caa gtc cgc agc agt tta nnn    1169
   Ser Lys His Pro Asn Trp Thr Asn Thr Gln Val Arg Ser Ser Leu Xaa
                   240                 245                 250
   aac acc act aca aaa ctt ggt gat tct ttc tac tat gga aaa ggg ctg    1217
   Asn Thr Thr Thr Lys Leu Gly Asp Ser Phe Tyr Tyr Gly Lys Gly Leu
               255                 260                 265
   atc aac gta cag gcg gca gct cag taa aacataaaaa accggccttg          1264
   Ile Asn Val Gln Ala Ala Ala Gln
           270                 275
   gccccgccgg tttttttatt tttcttcctc cgcatgttca atccgctcca taatcgacgg 1324
   atggctccct ctgaaaattt taacgagaaa cggcgggttg acccggctca gtcccgtaac 1384
   ggccaagtcc tgaaacgtct caatcgccgc ttcccggttt ccggtcagct caatgccgta 1444
   acggtcggcg gcgttttcct gataccggga gacggcattc gtaatcggat c          1495
<210> 2
   <211> 382
   <212> PRT
   <213> Bacillus amyloliquefaciens
<400> 2
   Xaa Arg Gly Lys Lys Val Trp Ile Ser Leu Leu Phe Ala Leu Ala Leu
   1 5 10 15
   Ile Phe Thr Met Ala Phe Gly Ser Thr Ser Ser Ala Gln Ala Ala Gly
   20 25 30
   Lys Ser Asn Gly Glu Lys Lys Tyr Ile Val Gly Phe Lys Gln Thr Met
   35 40 45
   Ser Thr Met Ser Ala Ala Lys Lys Lys Asp Val Ile Ser Glu Lys Gly
   50 55 60
   Gly Lys Val Gln Lys Gln Phe Lys Tyr Val Asp Ala Ala Ser Ala Thr
   65 70 75 80
   Leu Asn Glu Lys Ala Val Lys Glu Leu Lys Lys Asp Pro Ser Val Ala
   85 90 95
   Tyr Val Glu Glu Asp His Val Ala His Ala Tyr Ala Gln Ser Val Pro
   100 105 110
   Tyr Gly Val Ser Gln Ile Lys Ala Pro Ala Leu His Ser Gln Gly Tyr
   115 120 125
   Thr Gly Ser Asn Val Lys Val Ala Val Ile Asp Ser Gly Ile Asp Ser
   130 135 140
   Ser His Pro Asp Leu Lys Val Ala Gly Gly Ala Ser Met Val Pro Ser
   145 150 155 160
   Glu Thr Xaa Xaa Phe Gln Asp Xaa Asn Ser His Gly Thr His Val Ala
   165 170 175
   Gly Thr Val Ala Ala Leu Asn Asn Ser Ile Gly Val Leu Gly Val Ala
   180 185 190
   Pro Ser Xaa Xaa Leu Tyr Ala Val Lys Val Leu Gly Xaa Xaa Gly Ser
   195 200 205
   Gly Gln Tyr Ser Trp Ile Ile Asn Gly Ile Glu Trp Ala Ile Ala Asn
   210 215 220
   Asn Met Asp Val Ile Asn Met Ser Leu Gly Gly Pro Ser Gly Ser Ala
   225 230 235 240
   Ala Leu Lys Ala Ala Val Asp Lys Ala Val Ala Ser Gly Val Val Val
   245 250 255
   Val Ala Ala Ala Gly Asn Glu Gly Xaa Xaa Gly Ser Ser Ser Thr Val
   260 265 270
   Gly Tyr Pro Gly Lys Tyr Pro Ser Val Ile Ala Val Gly Ala Val Asp
   275 280 285
   Ser Ser Asn Gln Arg Ala Ser Phe Ser Ser Val Gly Pro Glu Leu Asp
   290 295 300
   Val Met Ala Pro Gly Val Ser Ile Gln Ser Thr Leu Pro Gly Asn Lys
   305 310 315 320
   Tyr Gly Ala Tyr Asn Gly Thr Ser Met Ala Ser Pro His Val Ala Gly
   325 330 335
   Ala Ala Ala Leu Ile Leu Ser Lys His Pro Asn Trp Thr Asn Thr Gln
   340 345 350
   Val Arg Ser Ser Leu Xaa Asn Thr Thr Thr Lys Leu Gly Asp Ser Phe
   355 360 365
   Tyr Tyr Gly Lys Gly Leu Ile Asn Val Gln Ala Ala Ala Gln
   370 375 380
<210> 3
   <211> 275
   <212> PRT
   <213> Bacillus amyloliquefaciens
<400> 3
<210> 4
   <211> 275
   <212> PRT
   <213> Bacillus subtilis
<400> 4
   Ala Gln Ser Val Pro Tyr Gly Ile Ser Gln Ile Lys Ala Pro Ala Leu
   1               5                   10                  15
   His Ser Gln Gly Tyr Thr Gly Ser Asn Val Lys Val Ala Val Ile Asp
               20                  25                  30
   Ser Gly Ile Asp Ser Ser His Pro Asp Leu Asn Val Arg Gly Gly Ala
           35                  40                  45
   Ser Phe Val Pro Ser Glu Thr Asn Pro Tyr Gln Asp Gly Ser Ser His
       50                  55                  60
   Gly Thr His Val Ala Gly Thr Ile Ala Ala Leu Asn Asn Ser Ile Gly
   65                  70                  75                  80
   Val Leu Gly Val Ser Pro Ser Ala Ser Leu Tyr Ala Val Lys Val Leu
                   85                  90                  95
   Asp Ser Thr Gly Ser Gly Gln Tyr Ser Trp Ile Ile Asn Gly Ile Glu
               100                 105                 110
   Trp Ala Ile Ser Asn Asn Met Asp Val Ile Asn Met Ser Leu Gly Gly
           115                 120                 125
   Pro Thr Gly Ser Thr Ala Leu Lys Thr Val Val Asp Lys Ala Val Ser
       130                 135                 140
   Ser Gly Ile Val Val Ala Ala Ala Ala Gly Asn Glu Gly Ser Ser Gly
   145                 150                 155                 160
   Ser Thr Ser Thr Val Gly Tyr Pro Ala Lys Tyr Pro Ser Thr Ile Ala
                   165                 170                 175
   Val Gly Ala Val Asn Ser Ser Asn Gln Arg Ala Ser Phe Ser Ser Ala
               180                 185                 190
   Gly Ser Glu Leu Asp Val Met Ala Pro Gly Val Ser Ile Gln Ser Thr
           195                 200                 205
   Leu Pro Gly Gly Thr Tyr Gly Ala Tyr Asn Gly Thr Ser Met Ala Thr
       210                 215                 220
   Pro His Val Ala Gly Ala Ala Ala Leu Ile Leu Ser Lys His Pro Thr
   225                 230                 235                 240
   Trp Thr Asn Ala Gln Val Arg Asp Arg Leu Glu Ser Thr Ala Thr Tyr
                   245                 250                 255
   Leu Gly Asn Ser Phe Tyr Tyr Gly Lys Gly Leu Ile Asn Val Gln Ala
               260                 265                 270
   Ala Ala Gln
           275
<210> 5
   <211> 274
   <212> PRT
   <213> Bacillus licheniformis
<400> 5
<210> 6
   <211> 269
   <212> PRT
   <213> Bacillus lentus
<400> 6
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 7
   <211> 15
   <212> PRT
   <213> Artificial Sequence
<220>
   <223> Description of Artificial Sequence: Synthetic
<400> 7
   Ile Lys Asp Phe His Val Tyr Phe Arg Glu Ser Arg Asp Ala Gly
   1 5 10 15
<210> 8
   <211> 15
   <212> PRT
   <213> Artificial Sequence
<220>
   <223> Description of Artificial Sequence: Synthetic
<400> 8
   Leu Glu Gln Ala Val Asn Ser Ala Thr Ser Arg Gly Val Leu Val
   1 5 10 15
<210> 9
   <211> 15
   <212> PRT
   <213> Artificial Sequence
<220>
   <223> Description of Artificial Sequence: Synthetic
<400> 9
   Ala Gln Ser Val Pro Trp Gly Ile Ser Arg Val Gln Ala Pro Ala
   1 5 10 15
<210> 10
   <211> 15
   <212> PRT
   <213> Artificial Sequence
<220>
   <223> Description of Artificial Sequence: Synthetic
<400> 10
   Val Pro Trp Gly Ile Ser Arg Val Gln Ala Pro Ala Ala His Asn
   1 5 10 15
<210> 11
   <211> 15
   <212> PRT
   <213> Artificial Sequence
<220>
   <223> Description of Artificial Sequence: Synthetic
<400> 11
   Gly Ile Ser Arg Val Gln Ala Pro Ala Ala His Asn Arg Gly Leu
    1               5                  10                  15
<210> 12
   <211> 15
   <212> PRT
   <213> Artificial Sequence
<220>
   <223> Description of Artificial Sequence: Synthetic
<400> 12
   Arg Val Gln Ala Pro Ala Ala His Asn Arg Gly Leu Thr Gly Ser
    1               5                  10                  15
<210> 13
   <211> 15
   <212> PRT
   <213> Artificial Sequence
<220>
   <223> Description of Artificial Sequence: Synthetic
<400> 13
   Ala Pro Ala Ala His Asn Arg Gly Leu Thr Gly Ser Gly Val Lys
    1               5                  10                  15
<210> 14
   <211> 15
   <212> PRT
   <213> Artificial Sequence
<220>
   <223> Description of Artificial Sequence: Synthetic
<400> 14
   Ala His Asn Arg Gly Leu Thr Gly Ser Gly Val Lys Val Ala Val
   1 5 10 15
<210> 15
   <211> 15
   <212> PRT
   <213> Artificial Sequence
<220>
   <223> Description of Artificial Sequence: Synthetic
<400> 15
   Arg Gly Leu Thr Gly Ser Gly Val Lys Val Ala Val Leu Asp Thr
   1 5 10 15
<210> 16
   <211> 15
   <212> PRT
   <213> Artificial Sequence
<220>
   <223> Description of Artificial Sequence: Synthetic
<400> 16
   Thr Gly Ser Gly Val Lys Val Ala Val Leu Asp Thr Gly Ile Ser
   1 5 10 15
<210> 17
   <211> 15
   <212> PRT
   <213> Artificial Sequence
<220>
   <223> Description of Artificial Sequence: Synthetic
<400> 17
   Gly Val Lys Val Ala Val Leu Asp Thr Gly Ile Ser Thr His Pro
   1 5 10 15
<210> 18
   <211> 15
   <212> PRT
   <213> Artificial Sequence
<220>
   <223> Description of Artificial Sequence: Synthetic
<400>18
   Val Ala Val Leu Asp Thr Gly Ile Ser Thr His Pro Asp Leu Asn
   1 5 10 15
<210> 19
   <211> 15
   <212> PRT
   <213> Artificial Sequence
<220>
   <223> Description of Artificial Sequence: Synthetic
<400> 19
   Leu Asp Thr Gly Ile Ser Thr His Pro Asp Leu Asn Ile Arg Gly
   1 5 10 15
<210> 20
   <211> 15
   <212> PRT
   <213> Artificial Sequence
<220>
   <223> Description of Artificial Sequence: Synthetic
<400> 20
   Gly Ile Ser Thr His Pro Asp Leu Asn Ile Arg Gly Gly Ala Ser
   1 5 10 15
<210> 21
   <211> 15
   <212> PRT
   <213> Artificial Sequence
<220>
   <223> Description of Artificial Sequence: Synthetic
<400> 21
   Thr His Pro Asp Leu Asn Ile Arg Gly Gly Ala Ser Phe Val Pro
   1 5 10 15
<210> 22
   <211> 15
   <212> PRT
   <213> Artificial Sequence
<220>
   <223> Description of Artificial Sequence: Synthetic
<400> 22
   Asp Leu Asn Ile Arg Gly Gly Ala Ser Phe Val Pro Gly Glu Pro
   1 5 10 15
<210> 23
   <211> 15
   <212> PRT
   <213> Artificial Sequence
<220>
   <223> Description of Artificial Sequence: Synthetic
<400> 23
   Ile Arg Gly Gly Ala Ser Phe Val Pro Gly Glu Pro Ser Thr Gln
   1 5 10 15
<210> 24
   <211> 15
   <212> PRT
   <213> Artificial Sequence
<220>
   <223> Description of Artificial Sequence: Synthetic
<400> 24
   Gly Ala Ser Phe Val Pro Gly Glu Pro Ser Thr Gln Asp Gly Asn
   1 5 10 15
<210> 25
   <211> 15
   <212> PRT
   <213> Artificial Sequence
<220>
   <223> Description of Artificial Sequence: Synthetic
<400> 25
   Phe Val Pro Gly Glu Pro Ser Thr Gln Asp Gly Asn Gly His Gly
   1 5 10 15
<210> 26
   <211> 15
   <212> PRT
   <213> Artificial Sequence
<220>
   <223> Description of Artificial Sequence: Synthetic
<400> 26
   Gly Glu Pro Ser Thr Gln Asp Gly Asn Gly His Gly Thr His Val
   1 5 10 15
<210> 27
   <211> 15
   <212> PRT
   <213> Artificial Sequence
<220>
   <223> Description of Artificial Sequence: Synthetic
<400> 27
   Ser Thr Gln Asp Gly Asn Gly His Gly Thr His Val Ala Gly Thr
   1 5 10 15
<210> 28
   <211> 15
   <212> PRT
   <213> Artificial Sequence
<220>
   <223> Description of Artificial Sequence: Synthetic
<400> 28
   Asp Gly Asn Gly His Gly Thr His Val Ala Gly Thr Ile Ala Ala
   1 5 10 15
<210> 29
   <211> 15
   <212> PRT
   <213> Artificial Sequence
<220>
   <223> Description of Artificial Sequence: Synthetic
<400> 29
   Gly His Gly Thr His Val Ala Gly Thr Ile Ala Ala Leu Asn Asn
   1 5 10 15
<210> 30
   <211> 15
   <212> PRT
   <213> Artificial Sequence
<220>
   <223> Description of Artificial Sequence: Synthetic
<400> 30
   Thr His Val Ala Gly Thr Ile Ala Ala Leu Asn Asn Ser Ile Gly
   1 5 10 15
<210> 31
   <211> 15
   <212> PRT
   <213> Artificial Sequence
<220>
   <223> Description of Artificial Sequence: Synthetic
<400> 31
   Ala Gly Thr Ile Ala Ala Leu Asn Asn Ser Ile Gly Val Leu Gly
   1 5 10 15
<210> 32
   <211> 15
   <212> PRT
   <213> Artificial Sequence
<220>
   <223> Description of Artificial Sequence: Synthetic
<400> 32
   Ile Ala Ala Leu Asn Asn Ser Ile Gly Val Leu Gly Val Ala Pro
   1 5 10 15
<210> 33
   <211> 15
   <212> PRT
   <213> Artificial Sequence
<220>
   <223> Description of Artificial Sequence: Synthetic
<400> 33
   Leu Asn Asn Ser Ile Gly Val Leu Gly Val Ala Pro Ser Ala Glu
   1 5 10 15
<210> 34
   <211> 15
   <212> PRT
   <213> Artificial Sequence
<220>
   <223> Description of Artificial Sequence: Synthetic
<400> 34
   Ser Ile Gly Val Leu Gly Val Ala Pro Ser Ala Glu Leu Tyr Ala
    1               5                  10                  15
<210> 35
   <211> 15
   <212> PRT
   <213> Artificial Sequence
<220>
   <223> Description of Artificial Sequence: Synthetic
<400> 35
   Val Leu Gly Val Ala Pro Ser Ala Glu Leu Tyr Ala Val Lys Val
    1               5                  10                  15
<210> 36
   <211> 15
   <212> PRT
   <213> Artificial Sequence
<220>
   <223> Description of Artificial Sequence: Synthetic
<400> 36
   Val Ala Pro Ser Ala Glu Leu Tyr Ala Val Lys Val Leu Gly Ala
    1               5                  10                  15
<210> 37
   <211> 15
   <212> PRT
   <213> Artificial Sequence
<220>
   <223> Description of Artificial Sequence: Synthetic
<400> 37
   Ser Ala Glu Leu Tyr Ala Val Lys Val Leu Gly Ala Ser Gly Ser
    1               5                  10                  15
<210> 38
   <211> 15
   <212> PRT
   <213> Artificial Sequence
<220>
   <223> Description of Artificial Sequence: Synthetic
<400> 38
   Leu Tyr Ala Val Lys Val Leu Gly Ala Ser Gly Ser Gly Ser Val
   1 5 10 15
<210> 39
   <211> 15
   <212> PRT
   <213> Artificial Sequence
<220>
   <223> Description of Artificial Sequence: Synthetic
<400> 39
   Val Lys Val Leu Gly Ala Ser Gly Ser Gly Ser Val Ser Ser Ile
   1 5 10 15
<210> 40
   <211> 15
   <212> PRT
   <213> Artificial Sequence
<220>
   <223> Description of Artificial Sequence: Synthetic
<400> 40
   Leu Gly Ala Ser Gly Ser Gly Ser Val Ser Ser Ile Ala Gln Gly
   1 5 10 15
<210> 41
   <211> 15
   <212> PRT
   <213> Artificial Sequence
<220>
   <223> Description of Artificial Sequence: Synthetic
<400> 41
   Ser Gly Ser Gly Ser Val Ser Ser Ile Ala Gln Gly Leu Glu Trp
   1 5 10 15
<210> 42
   <211> 15
   <212> PRT
   <213> Artificial Sequence
<220>
   <223> Description of Artificial Sequence: Synthetic
<400> 42
   Gly Ser Val Ser Ser Ile Ala Gln Gly Leu Glu Trp Ala Gly Asn
    1               5                  10                  15
<210> 43
   <211> 15
   <212> PRT
   <213> Artificial Sequence
<220>
   <223> Description of Artificial Sequence: Synthetic
<400> 43
   Ser Ser Ile Ala Gln Gly Leu Glu Trp Ala Gly Asn Asn Gly Met
    1               5                  10                  15
<210> 44
   <211> 15
   <212> PRT
   <213> Artificial Sequence
<220>
   <223> Description of Artificial Sequence: Synthetic
<400> 44
   Ala Gln Gly Leu Glu Trp Ala Gly Asn Asn Gly Met His Val Ala
    1               5                  10                  15
<210> 45
   <211> 15
   <212> PRT
   <213> Artificial Sequence
<220>
   <223> Description of Artificial Sequence: Synthetic
<400> 45
   Leu Glu Trp Ala Gly Asn Asn Gly Met His Val Ala Asn Leu Ser
    1               5                  10                  15
<210> 46
   <211> 15
   <212> PRT
   <213> Artificial Sequence
<220>
   <223> Description of Artificial Sequence: Synthetic
<400> 46
   Ala Gly Asn Asn Gly Met His Val Ala Asn Leu Ser Leu Gly Ser
   1               5                   10                  15
<210> 47
   <211> 15
   <212> PRT
   <213> Artificial Sequence
<220>
   <223> Description of Artificial Sequence: Synthetic
<400> 47
   Asn Gly Met His Val Ala Asn Leu Ser Leu Gly Ser Pro Ser Pro
   1               5                   10                  15
<210> 48
   <211> 15
   <212> PRT
   <213> Artificial Sequence
<220>
   <223> Description of Artificial Sequence: Synthetic
<400> 48
   His Val Ala Asn Leu Ser Leu Gly Ser Pro Ser Pro Ser Ala Thr
   1               5                   10                  15
<210> 49
   <211> 15
   <212> PRT
   <213> Artificial Sequence
<220>
   <223> Description of Artificial Sequence: Synthetic
<400> 49
   Asn Leu Ser Leu Gly Ser Pro Ser Pro Ser Ala Thr Leu Glu Gln
   1               5                   10                  15
<210> 50
   <211> 15
   <212> PRT
   <213> Artificial Sequence
<220>
   <223> Description of Artificial Sequence: Synthetic
<400> 50
   Leu Gly Ser Pro Ser Pro Ser Ala Thr Leu Glu Gln Ala Val Asn
   1 5 10 15
<210> 51
   <211> 15
   <212> PRT
   <213> Artificial Sequence
<220>
   <223> Description of Artificial Sequence: Synthetic
<400> 51
   Pro Ser Pro Ser Ala Thr Leu Glu Gln Ala Val Asn Ser Ala Thr
   1 5 10 15
<210> 52
   <211> 15
   <212> PRT
   <213> Artificial Sequence
<220>
   <223> Description of Artificial Sequence: Synthetic
<400> 52
   Ser Ala Thr Leu Glu Gln Ala Val Asn Ser Ala Thr Ser Arg Gly
   1 5 10 15
<210> 53
   <211> 15
   <212> PRT
   <213> Artificial Sequence
<220>
   <223> Description of Artificial Sequence: Synthetic
<400> 53
   Leu Glu Gln Ala Val Asn Ser Ala Thr Ser Arg Gly Val Leu Val
   1 5 10 15
<210> 54
   <211> 15
   <212> PRT
   <213> Artificial Sequence
<220>
   <223> Description of Artificial Sequence: Synthetic
<400> 54
   Ala Val Asn Ser Ala Thr Ser Arg Gly Val Leu Val Val Ala Ala
   1 5 10 15
<210> 55
   <211> 15
   <212> PRT
   <213> Artificial Sequence
<220>
   <223> Description of Artificial Sequence: Synthetic
<400> 55
   Ser Ala Thr Ser Arg Gly Val Leu Val Val Ala Ala Ser Gly Asn
   1 5 10 15
<210> 56
   <211> 15
   <212> PRT
   <213> Artificial Sequence
<220>
   <223> Description of Artificial Sequence: Synthetic
<400> 56
   Ser Arg Gly Val Leu Val Val Ala Ala Ser Gly Asn Ser Gly Ala
   1 5 10 15
<210> 57
   <211> 15
   <212> PRT
   <213> Artificial Sequence
<220>
   <223> Description of Artificial Sequence: Synthetic
<400> 57
   Val Leu Val Val Ala Ala Ser Gly Asn Ser Gly Ala Gly Ser Ile
   1 5 10 15
<210> 58
   <211> 15
   <212> PRT
   <213> Artificial Sequence
<220>
   <223> Description of Artificial Sequence: Synthetic
<400> 58
   Val Ala Ala Ser Gly Asn Ser Gly Ala Gly Ser Ile Ser Tyr Pro
   1 5 10 15
<210> 59
   <211> 15
   <212> PRT
   <213> Artificial Sequence
<220>
   <223> Description of Artificial Sequence: Synthetic
<400> 59
   Ser Gly Asn Ser Gly Ala Gly Ser Ile Ser Tyr Pro Ala Arg Tyr
   1 5 10 15
<210> 60
   <211> 15
   <212> PRT
   <213> Artificial Sequence
<220>
   <223> Description of Artificial Sequence: Synthetic
<400> 60
   Ser Gly Ala Gly Ser Ile Ser Tyr Pro Ala Arg Tyr Ala Asn Ala
   1 5 10 15
<210> 61
   <211> 15
   <212> PRT
   <213> Artificial Sequence
<220>
   <223> Description of Artificial Sequence: Synthetic
<400> 61
   Gly Ser Ile Ser Tyr Pro Ala Arg Tyr Ala Asn Ala Met Ala Val
   1 5 10 15
<210> 62
   <211> 15
   <212> PRT
   <213> Artificial Sequence
<220>
   <223> Description of Artificial Sequence: Synthetic
<400> 62
   Ser Tyr Pro Ala Arg Tyr Ala Asn Ala Met Ala Val Gly Ala Thr
   1 5 10 15
<210> 63
   <211> 15
   <212> PRT
   <213> Artificial Sequence
<220>
   <223> Description of Artificial Sequence: Synthetic
<400> 63
   Ala Arg Tyr Ala Asn Ala Met Ala Val Gly Ala Thr Asp Gln Asn
   1 5 10 15
<210> 64
   <211> 15
   <212> PRT
   <213> Artificial Sequence
<220>
   <223> Description of Artificial Sequence: Synthetic
<400> 64
   Ala Asn Ala Met Ala Val Gly Ala Thr Asp Gln Asn Asn Asn Arg
   1 5 10 15
<210> 65
   <211> 15
   <212> PRT
   <213> Artificial Sequence
<220>
   <223> Description of Artificial Sequence: Synthetic
<400> 65
   Met Ala Val Gly Ala Thr Asp Gln Asn Asn Asn Arg Ala Ser Phe
   1 5 10 15
<210> 66
   <211> 15
   <212> PRT
   <213> Artificial Sequence
<220>
   <223> Description of Artificial Sequence: Synthetic
<400> 66
   Gly Ala Thr Asp Gln Asn Asn Asn Arg Ala Ser Phe Ser Gln Tyr
    1               5                  10                  15
<210> 67
   <211> 15
   <212> PRT
   <213> Artificial Sequence
<220>
   <223> Description of Artificial Sequence: Synthetic
<400> 67
   Asp Gln Asn Asn Asn Arg Ala Ser Phe Ser Gln Tyr Gly Ala Gly
    1               5                  10                  15
<210> 68
   <211> 15
   <212> PRT
   <213> Artificial Sequence
<220>
   <223> Description of Artificial Sequence: Synthetic
<400> 68
   Asn Asn Arg Ala Ser Phe Ser Gln Tyr Gly Ala Gly Leu Asp Ile
    1               5                  10                  15
<210> 69
   <211> 15
   <212> PRT
   <213> Artificial Sequence
<220>
   <223> Description of Artificial Sequence: Synthetic
<400> 69
   Ala Ser Phe Ser Gln Tyr Gly Ala Gly Leu Asp Ile Val Ala Pro
    1               5                  10                  15
<210> 70
   <211> 15
   <212> PRT
   <213> Artificial Sequence
<220>
   <223> Description of Artificial Sequence: Synthetic
<400> 70
   Ser Gln Tyr Gly Ala Gly Leu Asp Ile Val Ala Pro Gly Val Asn
    1               5                  10                  15
<210> 71
   <211> 15
   <212> PRT
   <213> Artificial Sequence
<220>
   <223> Description of Artificial Sequence: Synthetic
<400> 71
   Gly Ala Gly Leu Asp Ile Val Ala Pro Gly Val Asn Val Gln Ser
    1               5                  10                  15
<210> 72
   <211> 15
   <212> PRT
   <213> Artificial Sequence
<220>
   <223> Description of Artificial Sequence: Synthetic
<400> 72
   Leu Asp Ile Val Ala Pro Gly Val Asn Val Gln Ser Thr Tyr Pro
    1               5                  10                  15
<210> 73
   <211> 15
   <212> PRT
   <213> Artificial Sequence
<220>
   <223> Description of Artificial Sequence: Synthetic
<400> 73
   Val Ala Pro Gly Val Asn Val Gln Ser Thr Tyr Pro Gly Ser Thr
    1               5                  10                  15
<210> 74
   <211> 15
   <212> PRT
   <213> Artificial Sequence
<220>
   <223> Description of Artificial Sequence: Synthetic
<400> 74
   Gly Val Asn Val Gln Ser Thr Tyr Pro Gly Ser Thr Tyr Ala Ser
   1 5 10 15
<210> 75
   <211> 15
   <212> PRT
   <213> Artificial Sequence
<220>
   <223> Description of Artificial Sequence: Synthetic
<400> 75
   Val Gln Ser Thr Tyr Pro Gly Ser Thr Tyr Ala Ser Leu Asn Gly
   1 5 10 15
<210> 76
   <211> 15
   <212> PRT
   <213> Artificial Sequence
<220>
   <223> Description of Artificial Sequence: Synthetic
<400> 76
   Thr Tyr Pro Gly Ser Thr Tyr Ala Ser Leu Asn Gly Thr Ser Met
   1 5 10 15
<210> 77
   <211> 15
   <212> PRT
   <213> Artificial Sequence
<220>
   <223> Description of Artificial Sequence: Synthetic
<400> 77
   Gly Ser Thr Tyr Ala Ser Leu Asn Gly Thr Ser Met Ala Thr Pro
   1 5 10 15
<210> 78
   <211> 15
   <212> PRT
   <213> Artificial Sequence
<220>
   <223> Description of Artificial Sequence: Synthetic
<400> 78
   Tyr Ala Ser Leu Asn Gly Thr Ser Met Ala Thr Pro His Val Ala
   1 5 10 15
<210> 79
   <211> 15
   <212> PRT
   <213> Artificial Sequence
<220>
   <223> Description of Artificial Sequence: Synthetic
<400> 79
   Leu Asn Gly Thr Ser Met Ala Thr Pro His Val Ala Gly Ala Ala
   1 5 10 15
<210> 80
   <211> 15
   <212> PRT
   <213> Artificial Sequence
<220>
   <223> Description of Artificial Sequence: Synthetic
<400> 80
   Thr Ser Met Ala Thr Pro His Val Ala Gly Ala Ala Ala Leu Val
   1 5 10 15
<210> 81
   <211> 15
   <212> PRT
   <213> Artificial Sequence
<220>
   <223> Description of Artificial Sequence: Synthetic
<400> 81
   Ala Thr Pro His Val Ala Gly Ala Ala Ala Leu Val Lys Gln Lys
   1 5 10 15
<210> 82
   <211> 15
   <212> PRT
   <213> Artificial Sequence
<220>
   <223> Description of Artificial Sequence: Synthetic
<400> 82
   Gly Val Ala Gly Ala Ala Ala Leu Val Lys Gln Lys Asn Pro Ser
   1               5                   10                  15
<210> 83
   <211> 15
   <212> PRT
   <213> Artificial Sequence
<220>
   <223> Description of Artificial Sequence: Synthetic
<400> 83
   Gly Ala Ala Ala Leu Val Lys Gln Lys Asn Pro Ser Trp Ser Asn
   1               5                   10                  15
<210> 84
   <211> 15
   <212> PRT
   <213> Artificial Sequence
<220>
   <223> Description of Artificial Sequence: Synthetic
<400> 84
   Ala Leu Val Lys Gln Lys Asn Pro Ser Trp Ser Asn Val Gln Ile
   1               5                   10                  15
<210> 85
   <211> 15
   <212> PRT
   <213> Artificial Sequence
<220>
   <223> Description of Artificial Sequence: Synthetic
<400> 85
   Lys Gln Lys Asn Pro Ser Trp Ser Val Asn Gln Ile Arg Asn His
   1               5                   10                  15
<210> 86
   <211> 15
   <212> PRT
   <213> Artificial Sequence
<220>
   <223> Description of Artificial Sequence: Synthetic
<400> 86
   Asn Pro Ser Trp Ser Asn Val Gln Ile Arg Asn His Leu Lys Asn
   1 5 10 15
<210> 87
   <211> 15
   <212> PRT
   <213> Artificial Sequence
<220>
   <223> Description of Artificial Sequence: Synthetic
<400> 87
   Trp Ser Asn Val Gln Ile Arg Asn His Leu Lys Asn Thr Ala Thr
   1 5 10 15
<210> 88
   <211> 15
   <212> PRT
   <213> Artificial Sequence
<220>
   <223> Description of Artificial Sequence: Synthetic
<400> 88
   Val Gln Ile Arg Asn His Leu Lys Asn Thr Ala Thr Ser Leu Gly
   1 5 10 15
<210> 89
   <211> 15
   <212> PRT
   <213> Artificial Sequence
<220>
   <223> Description of Artificial Sequence: Synthetic
<400> 89
   Arg Asn His Leu Lys Asn Thr Ala Thr Ser Leu Gly Ser Thr Asn
   1 5 10 15
<210> 90
   <211> 15
   <212> PRT
   <213> Artificial Sequence
<220>
   <223> Description of Artificial Sequence: Synthetic
<400> 90
   Leu Lys Asn Thr Ala Thr Ser Leu Gly Ser Thr Asn Leu Tyr Gly
    1               5                  10                  15
<210> 91
   <211> 15
   <212> PRT
   <213> Artificial Sequence
<220>
   <223> Description of Artificial Sequence: Synthetic
<400> 91
   Thr Ala Thr Ser Leu Gly Ser Thr Asn Leu Tyr Gly Ser Gly Leu
    1               5                  10                  15
<210> 92
   <211> 15
   <212> PRT
   <213> Artificial Sequence
<220>
   <223> Description of Artificial Sequence: Synthetic
<400> 92
   Ser Leu Gly Ser Thr Asn Leu Tyr Gly Ser Gly Leu Val Asn Ala
    1               5                  10                  15
<210> 93
   <211> 15
   <212> PRT
   <213> Artificial Sequence
<220>
   <223> Description of Artificial Sequence: Synthetic
<400> 93
   Ser Thr Asn Leu Tyr Gly Ser Gly Leu Val Asn Ala Glu Ala Ala
    1               5                  10                  15
<210> 94
   <211> 15
   <212> PRT
   <213> Artificial Sequence
<220>
   <223> Description of Artificial Sequence: Synthetic
<400> 94
   Asn Leu Tyr Gly Ser Gly Leu Val Asn Ala Glu Ala Ala Thr Arg
   1 5 10 15
<210> 95
   <211> 15
   <212> PRT
   <213> Artificial Sequence
<220>
   <223> Description of Artificial Sequence: Synthetic
<400> 95
   Asp Ala Glu Leu His Ile Phe Arg Val Phe Thr Asn Asn Gln Val
   1 5 10 15
<210> 96
   <211> 15
   <212> PRT
   <213> Artificial Sequence
<220>
   <223> Description of Artificial Sequence: Synthetic
<400> 96
   Pro Leu Arg Arg Ala Ser Leu Ser Leu Gly Ser Gly Phe Trp His
   1 5 10 15
<210> 97
   <211> 15
   <212> PRT
   <213> Artificial Sequence
<220>
   <223> Description of Artificial Sequence: Synthetic
<400> 97
   Arg Ala Ser Leu Ser Leu Gly Ser Gly Phe Trp His Ala Thr Gly
   1 5 10 15
<210> 98
   <211> 15
   <212> PRT
   <213> Artificial Sequence
<220>
   <223> Description of Artificial Sequence: Synthetic
<400> 98
   Leu Ser Leu Gly Ser Gly Phe Trp His Ala Thr Gly Arg His Ser
     1               5                  10                  15
<210> 99
   <211> 15
   <212> PRT
   <213> Artificial Sequence
<220>
   <223> Description of Artificial Sequence: Synthetic
<400> 99
   Gly Ser Gly Phe Trp His Ala Thr Gly Arg His Ser Ser Arg Arg
     1               5                  10                  15
<210> 100
   <211> 15
   <212> PRT
   <213> Artificial Sequence
<220>
   <223> Description of Artificial Sequence: Synthetic
<400> 100
   Phe Trp His Ala Thr Gly Arg His Ser Ser Arg Arg Leu Leu Arg
     1               5                  10                  15
<210> 101
   <211> 15
   <212> PRT
   <213> Artificial Sequence
<220>
   <223> Description of Artificial Sequence: Synthetic
<400> 101
   Ala Thr Gly Arg His Ser Ser Arg Arg Leu Leu Arg Ala Ile Pro
     1               5                  10                  15
<210> 102
   <211> 15
   <212> PRT
   <213> Artificial Sequence
<220>
   <223> Description of Artificial Sequence: Synthetic
<400> 102
   Arg His Ser Ser Arg Arg Leu Leu Arg Ala Ile Pro Arg Gln Val
   1 5 10 15
<210> 103
   <211> 15
   <212> PRT
   <213> Artificial Sequence
<220>
   <223> Description of Artificial Sequence: Synthetic
<400> 103
   Ser Arg Arg Leu Leu Arg Ala Ile Pro Arg Gln Val Ala Gln Thr
   1 5 10 15
<210> 104
   <211> 15
   <212> PRT
   <213> Artificial Sequence
<220>
   <223> Description of Artificial Sequence: Synthetic
<400> 104
   Leu Leu Arg Ala Ile Pro Arg Gln Val Ala Gln Thr Leu Gln Ala
   1 5 10 15
<210> 105
   <211> 15
   <212> PRT
   <213> Artificial Sequence
<220>
   <223> Description of Artificial Sequence: Synthetic
<400> 105
   Ala Ile Pro Arg Gln Val Ala Gln Thr Leu Gln Ala Asp Val Leu
   1 5 10 15
<210> 106
   <211> 15
   <212> PRT
   <213> Artificial Sequence
<220>
   <223> Description of Artificial Sequence: Synthetic
<400> 106
   Arg Gln Val Ala Gln Thr Leu Gln Ala Asp Val Leu Trp Gln Met
    1               5                  10                  15
<210> 107
   <211> 15
   <212> PRT
   <213> Artificial Sequence
<220>
   <223> Description of Artificial Sequence: Synthetic
<400> 107
   Ala Gln Thr Leu Gln Ala Asp Val Leu Trp Gln Met Gly Tyr Thr
    1               5                  10                  15
<210> 108
   <211> 15
   <212> PRT
   <213> Artificial Sequence
<220>
   <223> Description of Artificial Sequence: Synthetic
<400> 108
   Leu Gln Ala Asp Val Leu Trp Gln Met Gly Tyr Thr Gly Ala Asn
    1               5                  10                  15
<210> 109
   <211> 15
   <212> PRT
   <213> Artificial Sequence
<220>
   <223> Description of Artificial Sequence: Synthetic
<400> 109
   Asp Val Leu Trp Gln Met Gly Tyr Thr Gly Ala Asn Val Arg Val
    1               5                  10                  15
<210> 110
   <211> 15
   <212> PRT
   <213> Artificial Sequence
<220>
   <223> Description of Artificial Sequence: Synthetic
<400> 110
   Trp Gln Met Gly Tyr Thr Gly Ala Asn Val Arg Val Ala Val Phe
   1 5 10 15
<210> 111
   <211> 15
   <212> PRT
   <213> Artificial Sequence
<220>
   <223> Description of Artificial Sequence: Synthetic
<400> 111
   Gly Tyr Thr Gly Ala Asn Val Arg Val Ala Val Phe Asp Thr Gly
   1 5 10 15
<210> 112
   <211> 15
   <212> PRT
   <213> Artificial Sequence
<220>
   <223> Description of Artificial Sequence: Synthetic
<400> 112
   Gly Ala Asn Val Arg Val Ala Val Phe Asp Thr Gly Leu Ser Glu
   1 5 10 15
<210> 113
   <211> 15
   <212> PRT
   <213> Artificial Sequence
<220>
   <223> Description of Artificial Sequence: Synthetic
<400> 113
   Val Arg Val Ala Val Phe Asp Thr Gly Leu Ser Glu Lys His Pro
   1 5 10 15
<210> 114
   <211> 15
   <212> PRT
   <213> Artificial Sequence
<220>
   <223> Description of Artificial Sequence: Synthetic
<400> 114
   Ala Val Phe Asp Thr Gly Leu Ser Glu Lys His Pro His Phe Lys
    1               5                  10                  15
<210> 115
   <211> 15
   <212> PRT
   <213> Artificial Sequence
<220>
   <223> Description of Artificial Sequence: Synthetic
<400> 115
   Asp Thr Gly Leu Ser Glu Lys His Pro His Phe Lys Asn Val Lys
    1               5                  10                  15
<210> 116
   <211> 15
   <212> PRT
   <213> Artificial Sequence
<220>
   <223> Description of Artificial Sequence: Synthetic
<400> 116
   Leu Ser Glu Lys His Pro His Phe Lys Asn Val Lys Glu Arg Thr
    1               5                  10                  15
<210> 117
   <211> 15
   <212> PRT
   <213> Artificial Sequence
<220>
   <223> Description of Artificial Sequence: Synthetic
<400> 117
   Lys His Pro His Phe Lys Asn Val Lys Glu Arg Thr Asn Trp Thr
    1               5                  10                  15
<210> 118
   <211> 15
   <212> PRT
   <213> Artificial Sequence
<220>
   <223> Description of Artificial Sequence: Synthetic
<400> 118
   His Phe Lys Asn Val Lys Glu Arg Thr Asn Trp Thr Asn Glu Arg
   1 5 10 15
<210> 119
   <211> 15
   <212> PRT
   <213> Artificial Sequence
<220>
   <223> Description of Artificial Sequence: Synthetic
<400> 119
   Asn Val Lys Glu Arg Thr Asn Trp Thr Asn Glu Arg Thr Leu Asp
   1 5 10 15
<210> 120
   <211> 15
   <212> PRT
   <213> Artificial Sequence
<220>
   <223> Description of Artificial Sequence: Synthetic
<400> 120
   Glu Arg Thr Asn Trp Thr Asn Glu Arg Thr Leu Asp Asp Gly Leu
   1 5 10 15
<210> 121
   <211> 15
   <212> PRT
   <213> Artificial Sequence
<220>
   <223> Description of Artificial Sequence: Synthetic
<400> 121
   Asn Trp Thr Asn Glu Arg Thr Leu Asp Asp Gly Leu Gly His Gly
   1 5 10 15
<210> 122
   <211> 15
   <212> PRT
   <213> Artificial Sequence
<220>
   <223> Description of Artificial Sequence: Synthetic
<400> 122
   Asn Glu Arg Thr Leu Asp Asp Gly Leu Gly His Gly Thr Phe Val
    1               5                  10                  15
<210> 123
   <211> 15
   <212> PRT
   <213> Artificial Sequence
<220>
   <223> Description of Artificial Sequence: Synthetic
<400> 123
   Thr Leu Asp Asp Gly Leu Gly His Gly Thr Phe Val Ala Gly Val
    1               5                  10                  15
<210> 124
   <211> 15
   <212> PRT
   <213> Artificial Sequence
<220>
   <223> Description of Artificial Sequence: Synthetic
<400> 124
   Asp Gly Leu Gly His Gly Thr Phe Val Ala Gly Val Ile Ala Ser
    1               5                  10                  15
<210> 125
   <211> 15
   <212> PRT
   <213> Artificial Sequence
<220>
   <223> Description of Artificial Sequence: Synthetic
<400> 125
   Gly His Gly Thr Phe Val Ala Gly Val Ile Ala Ser Met Arg Glu
    1               5                  10                  15
<210> 126
   <211> 15
   <212> PRT
   <213> Artificial Sequence
<220>
   <223> Description of Artificial Sequence: Synthetic
<400> 126
   Thr Phe Val Ala Gly Val Ile Ala Ser Met Arg Glu Cys Gln Gly
   1               5                   10                  15
<210> 127
   <211> 15
   <212> PRT
   <213> Artificial Sequence
<220>
   <223> Description of Artificial Sequence: Synthetic
<400> 127
   Ala Gly Val Ile Ala Ser Met Arg Glu Cys Gln Gly Phe Ala Pro
   1               5                   10                  15
<210> 128
   <211> 15
   <212> PRT
   <213> Artificial Sequence
<220>
   <223> Description of Artificial Sequence: Synthetic
<400> 128
   Ile Ala Ser Met Arg Glu Cys Gln Gly Phe Ala Pro Asp Ala Glu
   1               5                   10                  15
<210> 129
   <211> 15
   <212> PRT
   <213> Artificial Sequence
<220>
   <223> Description of Artificial Sequence: Synthetic
<400> 129
   Met Arg Glu Cys Gln Gly Phe Ala Pro Asp Ala Glu Leu His Ile
   1               5                   10                  15
<210> 130
   <211> 15
   <212> PRT
   <213> Artificial Sequence
<220>
   <223> Description of Artificial Sequence: Synthetic
<400> 130
   Cys Gln Gly Phe Ala Pro Asp Ala Glu Leu His Ile Phe Arg Val
   1 5 10 15
<210> 131
   <211> 15
   <212> PRT
   <213> Artificial Sequence
<220>
   <223> Description of Artificial Sequence: Synthetic
<400> 131
   Phe Ala Pro Asp Ala Glu Leu His Ile Phe Arg Val Phe Thr Asn
   1 5 10 15
<210> 132
   <211> 15
   <212> PRT
   <213> Artificial Sequence
<220>
   <223> Description of Artificial Sequence: Synthetic
<400> 132
   Asp Ala Glu Leu His Ile Phe Arg Val Phe Thr Asn Asn Gln Val
   1 5 10 15
<210> 133
   <211> 15
   <212> PRT
   <213> Artificial Sequence
<220>
   <223> Description of Artificial Sequence: Synthetic
<400> 133
   Leu His Ile Phe Arg Val Phe Thr Asn Asn Gln Val Ser Tyr Thr
   1 5 10 15
<210> 134
   <211> 15
   <212> PRT
   <213> Artificial Sequence
<220>
   <223> Description of Artificial Sequence: Synthetic
<400> 134
   Phe Arg Val Phe Thr Asn Asn Gln Val Ser Tyr Thr Ser Trp Phe
   1 5 10 15
<210> 135
   <211> 15
   <212> PRT
   <213> Artificial Sequence
<220>
   <223> Description of Artificial Sequence: Synthetic
<400> 135
   Phe Thr Asn Asn Gln Val Ser Tyr Thr Ser Trp Phe Leu Asp Ala
   1 5 10 15
<210> 136
   <211> 15
   <212> PRT
   <213> Artificial Sequence
<220>
   <223> Description of Artificial Sequence: Synthetic
<400> 136
   Asn Gln Val Ser Tyr Thr Ser Trp Phe Leu Asp Ala Phe Asn Tyr
   1 5 10 15
<210> 137
   <211> 15
   <212> PRT
   <213> Artificial Sequence
<220>
   <223> Description of Artificial Sequence: Synthetic
<400> 137
   Ser Tyr Thr Ser Trp Phe Leu Asp Ala Phe Asn Tyr Ala Ile Leu
   1 5 10 15
<210> 138
   <211> 15
   <212> PRT
   <213> Artificial Sequence
<220>
   <223> Description of Artificial Sequence: Synthetic
<400> 138
   Ser Trp Phe Leu Asp Ala Phe Asn Tyr Ala Ile Leu Lys Lys Ile
   1 5 10 15
<210> 139
   <211> 15
   <212> PRT
   <213> Artificial Sequence
<220>
   <223> Description of Artificial Sequence: Synthetic
<400> 139
   Leu Asp Ala Phe Asn Tyr Ala Ile Leu Lys Lys Ile Asp Val Leu
   1 5 10 15
<210> 140
   <211> 15
   <212> PRT
   <213> Artificial Sequence
<220>
   <223> Description of Artificial Sequence: Synthetic
<400> 140
   Phe Asn Tyr Ala Ile Leu Lys Lys Ile Asp Val Leu Asn Leu Ser
   1 5 10 15
<210> 141
   <211> 15
   <212> PRT
   <213> Artificial Sequence
<220>
   <223> Description of Artificial Sequence: Synthetic
<400> 141
   Ala Ile Leu Lys Lys Ile Asp Val Leu Asn Leu Ser Ile Gly Gly
   1 5 10 15
<210> 142
   <211> 15
   <212> PRT
   <213> Artificial Sequence
<220>
   <223> Description of Artificial Sequence: Synthetic
<400> 142
   Lys Lys Ile Asp Val Leu Asn Leu Ser Ile Gly Gly Pro Asp Phe
   1 5 10 15
<210> 143
   <211> 15
   <212> PRT
   <213> Artificial Sequence
<220>
   <223> Description of Artificial Sequence: Synthetic
<400> 143
   Asp Val Leu Asn Leu Ser Ile Gly Gly Pro Asp Phe Met Asp His
   1 5 10 15
<210> 144
   <211> 15
   <212> PRT
   <213> Artificial Sequence
<220>
   <223> Description of Artificial Sequence: Synthetic
<400> 144
   Asn Leu Ser Ile Gly Gly Pro Asp Phe Met Asp His Pro Phe Val
   1 5 10 15
<210> 145
   <211> 15
   <212> PRT
   <213> Artificial Sequence
<220>
   <223> Description of Artificial Sequence: Synthetic
<400> 145
   Ile Gly Gly Pro Asp Phe Met Asp His Pro Phe Val Asp Lys Val
   1 5 10 15
<210> 146
   <211> 15
   <212> PRT
   <213> Artificial Sequence
<220>
   <223> Description of Artificial Sequence: Synthetic
<400> 146
   Pro Asp Phe Met Asp His Pro Phe Val Asp Lys Val Trp Glu Leu
   1 5 10 15
<210> 147
   <211> 15
   <212> PRT
   <213> Artificial Sequence
<220>
   <223> Description of Artificial Sequence: Synthetic
<400> 147
   Met Asp His Pro Phe Val Asp Lys Val Trp Glu Leu Thr Ala Asn
   1 5 10 15
<210> 148
   <211> 15
   <222> PRT
   <213> Artificial Sequence
<220>
   <223> Description of Artificial Sequence: Synthetic
<400> 148
   Pro Phe Val Asp Lys Val Trp Glu Leu Thr Ala Asn Asn Val Ile
   1 5 10 15
<210> 149
   <211> 15
   <212> PRT
   <213> Artificial Sequence
<220>
   <223> Description of Artificial Sequence: Synthetic
<400> 149
   Asp Lys Val Trp Glu Leu Thr Ala Asn Asn Val Ile Met Val Ser
   1 5 10 15
<210> 150
   <211> 15
   <212> PRT
   <213> Artificial Sequence
<220>
   <223> Description of Artificial Sequence: Synthetic
<400> 150
   Trp Glu Leu Thr Ala Asn Asn Val Ile Met Val Ser Ala Ile Gly
   1 5 10 15
<210> 151
   <211> 15
   <212> PRT
   <213> Artificial Sequence
<220>
   <223> Description of Artificial Sequence: Synthetic
<400> 151
   Thr Ala Asn Asn Val Ile Met Val Ser Ala Ile Gly Asn Asp Gly
   1 5 10 15
<210> 152
   <211> 15
   <212> PRT
   <213> Artificial Sequence
<220>
   <223> Description of Artificial Sequence: Synthetic
<400> 152
   Asn Val Ile Met Val Ser Ala Ile Gly Asn Asp Gly Pro Leu Tyr
   1 5 10 15
<210> 153
   <211> 15
   <212> PRT
   <213> Artificial Sequence
<220>
   <223> Description of Artificial Sequence: Synthetic
<400> 153
   Met Val Ser Ala Ile Gly Asn Asp Gly Pro Leu Tyr Gly Thr Ile
   1 5 10 15
<210> 154
   <211> 15
   <212> PRT
   <213> Artificial Sequence
<220>
   <223> Description of Artificial Sequence: Synthetic
<400> 154
   Ala Ile Gly Asn Asp Gly Pro Leu Tyr Gly Thr Leu Asn Asn Pro
    1               5                  10                  15
<210> 155
   <211> 15
   <212> PRT
   <213> Artificial Sequence
<220>
   <223> Description of Artificial Sequence: Synthetic
<400> 155
   Asn Asp Gly Pro Leu Tyr Gly Thr Leu Asn Asn Pro Ala Asp Gln
    1               5                  10                  15
<210> 156
   <211> 15
   <212> PRT
   <213> Artificial Sequence
<220>
   <223> Description of Artificial Sequence: Synthetic
<400> 156
   Pro Leu Tyr Gly Thr Leu Asn Asn Pro Ala Asp Gln Met Asp Val
    1               5                  10                  15
<210> 157
   <211> 15
   <212> PRT
   <213> Artificial Sequence
<220>
   <223> Description of Artificial Sequence: Synthetic
<400> 157
   Gly Thr Leu Asn Asn Pro Ala Asp Gln Met Asp Val Ile Gly Val
    1               5                  10                  15
<210> 158
   <211> 15
   <212> PRT
   <213> Artificial Sequence
<220>
   <223> Description of Artificial Sequence: Synthetic
<400> 158
   Asn Asn Pro Ala Asp Gln Met Asp Val Ile Gly Val Gly Gly Ile
    1               5                  10                  15
<210> 159
   <211> 15
   <212> PRT
   <213> Artificial Sequence
<220>
   <223> Description of Artificial Sequence: Synthetic
<400> 159
   Ala Asp Gln Met Asp Val Ile Gly Val Gly Gly Ile Asp Phe Glu
    1               5                  10                  15
<210> 160
   <211> 15
   <212> PRT
   <213> Artificial Sequence
<220>
   <223> Description of Artificial Sequence: Synthetic
<400> 160
   Met Asp Val Ile Gly Val Gly Gly Ile Asp Phe Glu Asp Asn Ile
    1               5                  10                  15
<210> 161
   <211> 15
   <212> PRT
   <213> Artificial Sequence
<220>
   <223> Description of Artificial Sequence: Synthetic
<400> 161
   Ile Gly Val Gly Gly Ile Asp Phe Glu Asp Asn Ile Ala Arg Phe
    1               5                  10                  15
<210> 162
   <211> 15
   <212> PRT
   <213> Artificial Sequence
<220>
   <223> Description of Artificial Sequence: Synthetic
<400> 162
   Gly Gly Ile Asp Phe Glu Asp Asn Ile Ala Arg Phe Ser Ser Arg
   1 5 10 15
<210> 163
   <211> 15
   <212> PRT
   <213> Artificial Sequence
<220>
   <223> Description of Artificial Sequence: Synthetic
<400> 163
   Asp Phe Glu Asp Asn Ile Ala Arg Phe Ser Ser Arg Gly Met Thr
   1 5 10 15
<210> 164
   <211> 15
   <212> PRT
   <213> Artificial Sequence
<220>
   <223> Description of Artificial Sequence: Synthetic
<400> 164
   Asp Asn Ile Ala Arg Phe Ser Ser Arg Gly Met Thr Thr Trp Glu
   1 5 10 15
<210> 165
   <211> 15
   <212> PRT
   <213> Artificial Sequence
<220>
   <223> Description of Artificial Sequence: Synthetic
<400> 165
   Ala Arg Phe Ser Ser Arg Gly Met Thr Thr Trp Glu Leu Pro Gly
   1 5 10 15
<210> 166
   <211> 15
   <212> PRT
   <213> Artificial Sequence
<220>
   <223> Description of Artificial Sequence: Synthetic
<400> 166
   Ser Ser Arg Gly Met Thr Thr Trp Glu Leu Pro Gly Gly Tyr Gly
   1 5 10 15
<210> 167
<211> 15
   <212> PRT
   <213> Artificial Sequence
<220>
   <223> Description of Artificial Sequence: Synthetic
<400> 167
   Gly Met Thr Thr Trp Glu Leu Pro Gly Gly Tyr Gly Arg Met Lys
   1 5 10 15
<210> 168
   <211> 15
   <212> PRT
   <213> Artificial Sequence
<220>
   <223> Description of Artificial Sequence: Synthetic
<400> 168
   Thr Trp Glu Leu Pro Gly Gly Tyr Gly Arg Met Lys Pro Asp Ile
   1 5 10 15
<210> 169
   <211> 15
   <212> PRT
   <213> Artificial Sequence
<220>
   <223> Description of Artificial Sequence: Synthetic
<400> 169
   Leu Pro Gly Gly Tyr Gly Arg Met Lys Pro Asp Ile Val Thr Tyr
   1 5 10 15
<210> 170
   <211> 15
   <212> PRT
   <213> Artificial Sequence
<220>
   <223> Description of Artificial Sequence: Synthetic
<400> 170
   Gly Tyr Gly Arg Met Lys Pro Asp Ile Val Thr Tyr Gly Ala Gly
   1 5 10 15
<210> 171
   <211> 15
   <212> PRT
   <213> Artificial Sequence
<220>
   <223> Description of Artificial Sequence: Synthetic
<400> 171
   Arg Met Lys Pro Asp Ile Val Thr Tyr Gly Ala Gly Val Arg Gly
   1 5 10 15
<210> 172
   <211> 15
   <212> PRT
   <213> Artificial Sequence
<220>
   <223> Description of Artificial Sequence: Synthetic
<400> 172
   Pro Asp Ile Val Thr Tyr Gly Ala Gly Val Arg Gly Ser Gly Val
   1 5 10 15
<210> 173
   <211> 15
   <212> PRT
   <213> Artificial Sequence
<220>
   <223> Description of Artificial Sequence: Synthetic
<400> 173
   Val Thr Tyr Gly Ala Gly Val Arg Gly Ser Gly Val Lys Gly Gly
   1 5 10 15
<210> 174
   <211> 15
   <212> PRT
   <213> Artificial Sequence
<220>
   <223> Description of Artificial Sequence: Synthetic
<400> 174
   Gly Ala Gly Val Arg Gly Ser Gly Val Lys Gly Gly Cys Arg Ala
   1 5 10 15
<210> 175
   <211> 15
   <212> PRT
   <213> Artificial Sequence
<220>
   <223> Description of Artificial Sequence: Synthetic
<400> 175
   Val Arg Gly Ser Gly Val Lys Gly Gly Cys Arg Ala Leu Ser Gly
   1 5 10 15
<210> 176
   <211> 15
   <212> PRT
   <213> Artificial Sequence
<220>
   <223> Description of Artificial Sequence: Synthetic
<400> 176
   Ser Gly Val Lys Gly Gly Cys Arg Ala Leu Ser Gly Thr Ser Val
   1 5 10 15
<210> 177
   <211> 15
   <212> PRT
   <213> Artificial Sequence
<220>
   <223> Description of Artificial Sequence: Synthetic
<400> 177
   Lys Gly Gly Cys Arg Ala Leu Ser Gly Thr Ser Val Ala Ser Pro
   1               5                   10                  15
<210> 178
   <211> 15
   <212> PRT
   <213> Artificial Sequence
<220>
   <223> Description of Artificial Sequence: Synthetic
<400> 178
   Cys Arg Ala Leu Ser Gly Thr Ser Val Ala Ser Pro Val Val Ala
   1               5                   10                  15
<210> 179
   <211> 15
   <212> PRT
   <213> Artificial Sequence
<220>
   <223> Description of Artificial Sequence: Synthetic
<400> 179
   Leu Ser Gly Thr Ser Val Ala Ser Pro Val Val Ala Gly Ala Val
   1               5                   10                  15
<210> 180
   <211> 15
   <212> PRT
   <213> Artificial Sequence
<220>
<223> Description of Artificial Sequence: Synthetic
<400> 180
   Thr Ser Val Ala Ser Pro Val Val Ala Gly Ala Val Thr Leu Leu
   1               5                   10                  15
<210> 181
   <211> 15
   <212> PRT
   <213> Artificial Sequence
<220>
   <223> Description of Artificial Sequence: Synthetic
<400> 181
   Ala Ser Pro Val Val Ala Gly Ala Val Thr Leu Leu Val Ser Thr
   1 5 10 15
<210> 182
   <211> 15
   <212> PRT
   <213> Artificial Sequence
<220>
   <223> Description of Artificial Sequence: Synthetic
<400> 182
   Val Val Ala Gly Ala Val Thr Leu Leu Val Ser Thr Val Gln Lys
   1 5 10 15
<210> 183
   <211> 15
   <212> PRT
   <213> Artificial Sequence
<220>
   <223> Description of Artificial Sequence: Synthetic
<400> 183
   Gly Ala Val Thr Leu Leu Val Ser Thr Val Gln Lys Arg Glu Leu
   1 5 10 15
<210> 184
   <211> 15
   <212> PRT
   <213> Artificial Sequence
<220>
   <223> Description of Artificial Sequence: Synthetic
<400> 184
   Thr Leu Leu Val Ser Thr Val Gln Lys Arg Glu Leu Val Asn Pro
   1 5 10 15
<210> 185
   <211> 15
   <212> PRT
   <213> Artificial Sequence
<220>
   <223> Description of Artificial Sequence: Synthetic
<400> 185
   Val Ser Thr Val Gln Lys Arg Glu Leu Val Asn Pro Ala Ser Met
   1 5 10 15
<210> 186
   <211> 15
   <212> PRT
   <213> Artificial Sequence
<220>
   <223> Description of Artificial Sequence: Synthetic
<400> 186
   Val Gln Lys Arg Glu Leu Val Asn Pro Ala Ser Met Lys Gln Ala
   1 5 10 15
<210> 187
   <211> 15
   <212> PRT
   <213> Artificial Sequence
<220>
   <223> Description of Artificial Sequence: Synthetic
<400> 187
   Arg Glu Leu Val Asn Pro Ala Ser Met Lys Gln Ala Leu Ile Ala
   1 5 10 15
<210> 188
   <211> 15
   <212> PRT
   <213> Artificial Sequence
<220>
   <223> Description of Artificial Sequence: Synthetic
<400> 188
   Val Asn Pro Ala Ser Met Lys Gln Ala Leu Ile Ala Ser Ala Arg
   1 5 10 15
<210> 189
   <211> 15
   <212> PRT
   <213> Artificial Sequence
<220>
   <223> Description of Artificial Sequence: Synthetic
<400> 189
   Ala Ser Met Lys Gln Ala Leu Ile Ala Ser Ala Arg Arg Leu Pro
    1               5                  10                  15
<210> 190
   <211> 15
   <212> PRT
   <213> Artificial Sequence
<220>
   <223> Description of Artificial Sequence: Synthetic
<400> 190
   Lys Gln Ala Leu Ile Ala Ser Ala Arg Arg Leu Pro Gly Val Asn
    1               5                  10                  15
<210> 191
   <211> 15
   <212> PRT
   <213> Artificial Sequence
<220>
   <223> Description of Artificial Sequence: Synthetic
<400> 191
   Leu Ile Ala Ser Ala Arg Arg Leu Pro Gly Val Asn Met Phe Glu
    1               5                  10                  15
<210> 192
   <211> 15
   <212> PRT
   <213> Artificial Sequence
<220>
   <223> Description of Artificial Sequence: Synthetic
<400> 192
   Ser Ala Arg Arg Leu Pro Gly Val Asn Met Phe Glu Gln Gly His
    1               5                  10                  15
<210> 193
   <211> 15
   <212> PRT
   <213> Artificial Sequence
<220>
   <223> Description of Artificial Sequence: Synthetic
<400> 193
   Arg Leu Pro Gly Val Asn Met Phe Glu Gln Gly His Gly Lys Leu
   1               5                   10                  15
<210> 194
   <211> 15
   <212> PRT
   <213> Artificial Sequence
<220>
   <223> Description of Artificial Sequence: Synthetic
<400> 194
   Gly Val Asn Met Phe Glu Gln Gly His Gly Lys Leu Asp Leu Leu
   1               5                   10                  15
<210> 195
   <211> 15
   <212> PRT
   <213> Artificial Sequence
<220>
   <223> Description of Artificial Sequence: Synthetic
<400> 195
   Met Phe Glu Gln Gly His Gly Lys Leu Asp Leu Leu Arg Ala Tyr
   1               5                   10                  15
<210> 196
   <211> 15
   <212> PRT
   <213> Artificial Sequence
<220>
   <223> Description of Artificial Sequence: Synthetic
<400> 196
   Gln Gly His Gly Lys Leu Asp Leu Leu Arg Ala Tyr Gln Ile Leu
   1               5                   10                  15
<210> 197
   <211> 15
   <212> PRT
   <213> Artificial Sequence
<220>
   <223> Description of Artificial Sequence: Synthetic
<400> 197
   Gly Lys Leu Asp Leu Leu Arg Ala Tyr Gln Ile Leu Asn Ser Tyr
   1 5 10 15
<210> 198
   <211> 15
   <212> PRT
   <213> Artificial Sequence
<220>
   <223> Description of Artificial Sequence: Synthetic
<400> 198
   Asp Leu Leu Arg Ala Tyr Gln Ile Leu Asn Ser Tyr Lys Pro Gln
   1 5 10 15
<210> 199
   <211> 15
   <212> PRT
   <213> Artificial Sequence
<220>
   <223> Description of Artificial Sequence: Synthetic
<400> 199
   Arg Ala Tyr Gln Ile Leu Asn Ser Tyr Lys Pro Gln Ala Ser Leu
   1 5 10 15
<210> 200
   <211> 15
   <212> PRT
   <213> Artificial Sequence
<220>
   <223> Description of Artificial Sequence: Synthetic
<400> 200
   Gln Ile Leu Asn Ser Tyr Lys Pro Gln Ala Ser Leu Ser Pro Ser
   1 5 10 15
<210> 201
   <211> 15
   <212> PRT
   <213> Artificial Sequence
<220>
   <223> Description of Artificial Sequence: Synthetic
<400> 201
   Asn Ser Tyr Lys Pro Gln Ala Ser Leu Ser Pro Ser Tyr Ile Asp
   1 5 10 15
<210> 202
   <211> 15
   <212> PRT
   <213> Artificial Sequence
<220>
   <223> Description of Artificial Sequence: Synthetic
<400> 202
   Lys Pro Gln Ala Ser Leu Ser Pro Ser Tyr Ile Asp Leu Thr Glu
   1 5 10 15
<210> 203
   <211> 15
   <212> PRT
   <213> Artificial Sequence
<220>
   <223> Description of Artificial Sequence: Synthetic
<400> 203
   Ala Ser Leu Ser Pro Ser Tyr Ile Asp Leu Thr Glu Cys Pro Tyr
   1 5 10 15
<210> 204
   <211> 15
   <212> PRT
   <213> Artificial Sequence
<220>
   <223> Description of Artificial Sequence: Synthetic
<400> 204
   Ser Pro Ser Tyr Ile Asp Leu Thr Glu Cys Pro Tyr Met Trp Pro
   1 5 10 15
<210> 205
   <211> 15
   <212> PRT
   <213> Artificial Sequence
<220>
   <223> Description of Artificial Sequence: Synthetic
<400> 205
   Tyr Ile Asp Leu Thr Glu Cys Pro Tyr Met Trp Pro Tyr Cys Ser
   1 5 10 15
<210> 206
   <211> 15
   <212> PRT
   <213> Artificial Sequence
<220>
   <223> Description of Artificial Sequence: Synthetic
<400> 206
   Leu Thr Glu Cys Pro Tyr Met Trp Pro Tyr Cys Ser Gln Pro Ile
   1 5 10 15
<210> 207
   <211> 15
   <212> PRT
   <213> Artificial Sequence
<220>
   <223> Description of Artificial Sequence: Synthetic
<400> 207
   Cys Pro Tyr Met Trp Pro Tyr Cys Ser Gln Pro Ile Tyr Tyr Gly
   1 5 10 15
<210> 208
   <211> 1052
   <212> PRT
   <213> Homo sapiens
<400> 208
<210> 209
   <211> 280
   <212> PRT
   <213> Homo sapiens
<400> 209
   Arg Ala Ile Pro Arg Gln Val Ala Gln Thr Leu Gln Ala Asp Val Leu
   1 5 10 15
   Trp Gln Met Gly Tyr Thr Gly Ala Asn Val Arg Val Ala Val Phe Asp
   20 25 30
   Thr Gly Leu Ser Glu Lys His Pro His Phe Lys Asn Val Lys Glu Arg
   35 40 45
   Thr Asn Trp Thr Asn Glu Arg Thr Leu Asp Asp Gly Leu Gly His Gly
   50 55 60
   Thr Phe Val Ala Gly Val Ile Ala Ser Met Arg Glu Cys Gln Gly Phe
   65 70 75 80
   Ala Pro Asp Ala Glu Leu His Ile Phe Arg Val Phe Thr Asn Asn Gln
   85 90 95
   Val Ser Tyr Thr Ser Trp Phe Leu Asp Ala Phe Asn Tyr Ala Ile Leu
   100 105 110
   Lys Lys Ile Asp Val Leu Asn Leu Ser Ile Gly Gly Pro Asp Phe Met
   115 120 125
   Asp His Pro Phe Val Asp Lys Val Trp Glu Leu Thr Ala Asn Asn Val
   130 135 140
   Ile Met Val Ser Ala Ile Gly Asn Asp Gly Pro Leu Tyr Gly Thr Leu
   145 150 155 160
   Asn Asn Pro Ala Asp Gln Met Asp Val Ile Gly Val Gly Gly Ile Asp
   165 170 175
   Phe Glu Asp Asn Ile Ala Arg Phe Ser Ser Arg Gly Met Thr Thr Trp
   180 185 190
   Glu Leu Pro Gly Gly Tyr Gly Arg Met Lys Pro Asp Ile Val Thr Tyr
   195 200 205
   Gly Ala Gly Val Arg Gly Ser Gly Val Lys Gly Gly Cys Arg Ala Leu
   210 215 220
   Ser Gly Thr Ser Val Ala Ser Pro Val Val Ala Gly Ala Val Thr Leu
   225 230 235 240
   Leu Val Ser Thr Val Gln Lys Arg Glu Leu Val Asn Pro Ala Ser Met
   245 250 255
   Lys Gln Ala Leu Ile Ala Ser Ala Arg Arg Leu Pro Gly Val Asn Met
   260 265 270
   Phe Glu Gln Gly His Gly Lys Leu
   275 280
<210> 210
   <211> 15
   <212> PRT
   <213> Artificial Sequence
<220>
   <223> Description of Artificial Sequence: Synthetic
<400> 210
   Gly Ser Ile Ser Tyr Pro Ala Arg Tyr Ala Asn Ala Met Ala Val
   1 5 10 15
<210> 211
<211> 15
   <212> PRT
   <213> Artificial Sequence
<220>
   <223> Description of Artificial Sequence: Synthetic
<400> 211
   Ala Ser Ile Ser Tyr Pro Ala Arg Tyr Ala Asn Ala Met Ala Val
   1               5                   10                  15
<210> 212
   <211> 15
   <212> PRT
   <213> Artificial Sequence
<220>
   <223> Description of Artificial Sequence: Synthetic
<400> 212
   Gly Ala Ile Ser Tyr Pro Ala Arg Tyr Ala Asn Ala Met Ala Val
   1               5                   10                  15
<210> 213
   <211> 15
   <212> PRT
   <213> Artificial Sequence
<220>
   <223> Description of Artificial Sequence: Synthetic
<400> 213
   Gly Ser Ala Ser Tyr Pro Ala Arg Tyr Ala Asn Ala Met Ala Val
   1               5                   10                  15
<210> 214
   <211> 15
   <212> PRT
   <213> Artificial Sequence
<220>
   <223> Description of Artificial Sequence: Synthetic
<400> 214
   Gly Ser Ile Ala Tyr Pro Ala Arg Tyr Ala Asn Ala Met Ala Val
   1               5                   10                  15
<210> 215
   <211> 15
   <212> PRT
   <213> Artificial Sequence
<220>
   <223> Description of Artificial Sequence: Synthetic
<400> 215
   Gly Ser Ile Ser Ala Pro Ala Arg Tyr Ala Asn Ala Met Ala Val
    1               5                   10                  15
<210> 216
   <211> 15
   <212> PRT
   <213> Artificial Sequence
<220>
   <223> Description of Artificial Sequence: Synthetic
<400> 216
   Gly Ser Ile Ser Tyr Ala Ala Arg Tyr Ala Asn Ala Met Ala Val
    1               5                   10                  15
<210> 217
   <211> 15
   <212> PRT
   <213> Artificial Sequence
<220>
   <223> Description of Artificial Sequence: Synthetic
<400> 217
   Gly Ser Ile Ser Tyr Pro Ala Ala Tyr Ala Asn Ala Met Ala Val
    1               5                   10                  15
<210> 218
   <211> 15
   <212> PRT
   <213> Artificial Sequence
<220>
   <223> Description of Artificial Sequence: Synthetic
<400> 218
   Gly Ser Ile Ser Tyr Pro Ala Arg Ala Ala Asn Ala Met Ala Val
   1 5 10 15
<210> 219
   <211> 15
   <212> PRT
   <213> Artificial Sequence
<220>
   <223> Description of Artificial Sequence: Synthetic
<400> 219
   Gly Ser Ile Ser Tyr Pro Ala Arg Tyr Ala Ala Ala Met Ala Val
   1 5 10 15
<210> 220
   <211> 15
   <212> PRT
   <213> Artificial Sequence
<220>
   <223> Description of Artificial Sequence: Synthetic
<400> 220
   Gly Ser Ile Ser Tyr Pro Ala Arg Tyr Ala Asn Ala Ala Ala Val
   1 5 10 15
<210> 221
   <211> 15
   <212> PRT
   <213> Artificial Sequence
<220>
   <223> Description of Artificial Sequence: Synthetic
<400> 221
   Gly Ser Ile Ser Tyr Pro Ala Arg Tyr Ala Asn Ala Met Ala Ala
   1 5 10 15
<210> 222
   <211> 15
   <212> PRT
   <213> Humicola insolens
<400> 222
   Pro Gly Gly Val Ala Tyr Ser Cys Ala Asp Gln Thr Pro Trp Ala
    1               5                  10                  15
<210> 223
   <211> 15
   <212> PRT
   <213> Humicola insolens
<400> 223
   Cys Gly Trp Ala Lys Lys Ala Pro Val Asn Gln Pro Val Phe Ser
    1               5                  10                  15
<210> 224
   <211> 276
   <212> PRT
   <213> Humicola insolens
<400> 224
   Met Arg Ser Ser Pro Leu Leu Pro Ser Ala Val Val Ala Ala Leu Pro
    1               5                  10                  15
   Val Leu Ala Leu Ala Ala Asp Gly Arg Ser Thr Arg Tyr Trp Asp Cys
               20                  25                  30
   Cys Lys Pro Ser Cys Gly Trp Ala Lys Lys Ala Pro Val Asn Gln Pro
   35 40 45
   Val Phe Ser Cys Asn Ala Asn Phe Gln Arg Ile Thr Asp Phe Asp Ala
   50 55 60
   Lys Ser Gly Cys Glu Pro Gly Gly Val Ala Tyr Ser Cys Ala Asp Gln
   65 70 75 80
   Thr Pro Trp Ala Val Asn Asp Asp Phe Ala Leu Gly Phe Ala Ala Thr
   85 90 95
   Ser Ile Ala Gly Ser Asn Glu Ala Gly Trp Cys Cys Ala Cys Tyr Glu
   100 105 110
   Leu Thr Phe Thr Ser Gly Pro Val Ala Gly Lys Lys Met Val Val Gln
   115 120 125
   Ser Thr Ser Thr Gly Gly Asp Leu Gly Ser Asn His Phe Asp Leu Asn
   130 135 140
   Ile Pro Gly Gly Gly Val Gly Ile Phe Asp Gly Cys Thr Pro Gln Phe
   145 150 155 160
   Gly Gly Leu Pro Gly Gln Arg Tyr Gly Gly Ile Ser Ser Arg Asn Glu
   165 170 175
   Cys Asp Arg Phe Pro Asp Ala Leu Lys Pro Gly Cys Tyr Trp Arg Phe
   180 185 190
   Asp Trp Phe Lys Asn Ala Asp Asn Pro Ser Phe Ser Phe Arg Gln Val
   195 200 205
   Gln Cys Pro Ala Glu Leu Val Ala Arg Thr Gly Cys Arg Arg Asn Asp
   210 215 220
   Asp Gly Asn Phe Pro Ala Val Gln Ile Pro Ser Ser Ser Thr Ser Ser
   225 230 235 240
   Pro Val Asn Gln Pro Thr Ser Thr Ser Thr Thr Ser Thr Ser Thr Thr
   245 250 255
   Ser Ser Pro Pro Val Gln Pro Thr Thr Pro Ser Gly Cys Thr Ala Glu
   260 265 270
   Arg Trp Ala Gln
   275
<210> 225
   <211> 18
   <212> PRT
   <213> Thermomyces lanuginosus
<400> 225
   Gly Asp Val Thr Gly Phe Leu Ala Leu Asp Asn Thr Asn Lys Leu Ile
   1 5 10 15
   Val Leu
<210> 226
   <211> 15
   <212> PRT
   <213> Thermomyces lanuginosus
<400> 226
   Ser Ile Glu Asn Trp Ile Gly Asn Leu Asn Phe Asp Leu Lys Glu
   1 5 10 15
<210> 227
   <211> 291
   <212> PRT
   <213> Thermomyces lanuginosus
<400> 227
   Met Arg Ser Ser Leu Val Leu Phe Phe Val Ser Ala Trp Thr Ala Leu
   1 5 10 15
   Ala Ser Pro Ile Arg Arg Glu Val Ser Gln Asp Leu Phe Asn Gln Phe
   20 25 30
   Asn Leu Phe Ala Gln Tyr Ser Ala Ala Ala Tyr Cys Gly Lys Asn Asn
   35 40 45
   Asp Ala Pro Ala Gly Thr Asn Ile Thr Cys Thr Gly Asn Ala Cys Pro
   50 55 60
   Glu Val Glu Lys Ala Asp Ala Thr Phe Leu Tyr Ser Phe Glu Asp Ser
   65 70 75 80
   Gly Val Gly Asp Val Thr Gly Phe Leu Ala Leu Asp Asn Thr Asn Lys
   85 90 95
   Leu Ile Val Leu Ser Phe Arg Gly Ser Arg Ser Ile Glu Asn Trp Ile
   100 105 110
   Gly Asn Leu Asn Phe Asp Leu Lys Glu Ile Asn Asp Ile Cys Ser Gly
   115 120 125
   Cys Arg Gly His Asp Gly Phe Thr Ser Ser Trp Arg Ser Val Ala Asp
   130 135 140
   Thr Leu Arg Gln Lys Val Glu Asp Ala Val Arg Glu His Pro Asp Tyr
   145 150 155 160
   Arg Val Val Phe Thr Gly His Ser Leu Gly Gly Ala Leu Ala Thr Val
   165 170 175
   Ala Gly Ala Asp Leu Arg Gly Asn Gly Tyr Asp Ile Asp Val Phe Ser
   180 185 190
   Tyr Gly Ala Pro Arg Val Gly Asn Arg Ala Phe Ala Glu Phe Leu Thr
   195 200 205
   Val Gln Thr Gly Gly Thr Leu Tyr Arg Ile Thr His Thr Asn Asp Ile
   210 215 220
   Val Pro Arg Leu Pro Pro Arg Glu Phe Gly Tyr Ser His Ser Ser Pro
   225 230 235 240
   Glu Tyr Trp Ile Lys Ser Gly Thr Leu Val Pro Val Thr Arg Asn Asp
   245 250 255
   Ile Val Lys Ile Glu Gly Ile Asp Ala Thr Gly Gly Asn Asn Gln Pro
   260 265 270
   Asn Ile Pro Asp Ile Pro Ala His Leu Trp Tyr Phe Gly Leu Ile Gly
   275 280 285
   Thr Cys Leu
   290
<210> 228
   <211> 15
   <212> PRT
   <213> Streptomyces plicatus
<400> 228
   Ile Lys Val Leu Leu Ser Val Leu Gly Asn His Gln Gly Ala Gly
   1               5                   10                  15
<210> 229
   <211> 313
   <212> PRT
   <213> Streptomyces plicatus
<400> 229
   Met Phe Thr Pro Val Arg Arg Arg Val Arg Thr Ala Ala Leu Ala Leu
   1               5                   10                  15
   Ser Ala Ala Ala Ala Leu Val Leu Gly Ser Thr Ala Ala Ser Gly Ala
               20                  25                  30
   Ser Ala Thr Pro Ser Pro Ala Pro Ala Pro Ala Pro Ala Pro Val Lys
           35                  40                  45
   Gln Gly Pro Thr Ser Val Ala Tyr Val Glu Val Asn Asn Asn Ser Met
       50                  55                  60
   Leu Asn Val Gly Lys Tyr Thr Leu Ala Asp Gly Gly Gly Asn Ala Phe
   65                  70                  75                  80
   Asp Val Ala Val Ile Phe Ala Ala Asn Ile Asn Tyr Asp Thr Gly Thr
                   85                  90                  95
   Lys Thr Ala Tyr Leu His Phe Asn Glu Asn Val Gln Arg Val Leu Asp
               100                 105                 110
   Asn Ala Val Thr Gln Ile Arg Pro Leu Gln Gln Gln Gly Ile Lys Val
                   115                 120                 125
   Leu Leu Ser Val Leu Gly Asn His Gln Gly Ala Gly Phe Ala Asn Phe
       130                 135                 140
   Pro Ser Gln Gln Ala Ala Ser Ala Phe Ala Lys Gln Leu Ser Asp Ala
   145                 150                 155                 160
   Val Ala Lys Tyr Gly Leu Asp Gly Val Asp Phe Asp Asp Glu Tyr Ala
                   165                 170                 175
   Glu Tyr Gly Asn Asn Gly Thr Ala Gln Pro Asn Asp Ser Ser Phe Val
               180                 185                 190
   His Leu Val Thr Ala Leu Arg Ala Asn Met Pro Asp Lys Ile Ile Ser
           195                 200                 205
   Leu Tyr Asn Ile Gly Pro Ala Ala Ser Arg Leu Ser Tyr Gly Gly Val
       210                 215                 220
   Asp Val Ser Asp Lys Phe Asp Tyr Ala Trp Asn Pro Tyr Tyr Gly Thr
   225                 230                 235                 240
   Trp Gln Val Pro Gly Ile Ala Leu Pro Lys Ala Gln Leu Ser Pro Ala
                   245                 250                 255
   Ala Val Glu Ile Gly Arg Thr Ser Arg Ser Thr Val Ala Asp Leu Ala
               260                 265                 270
   Arg Arg Thr Val Asp Glu Gly Tyr Gly Val Tyr Leu Thr Tyr Asn Leu
           275                 280                 285
   Asp Gly Gly Asp Arg Thr Ala Asp Val Ser Ala Phe Thr Arg Glu Leu
       290                 295                 300
   Tyr Gly Ser Glu Ala Val Arg Thr Pro
   305                 310
<210> 230
   <211> 15
   <212> PRT
   <213> Bacillus amyloliquefaciens
<400> 230
   Gly Thr Val Ala Ala Leu Asn Asn Ser Ile Gly Val Leu Gly Val
   1               5                   10                  15
<210> 231
   <211> 15
   <212> PRT
   <213> Bacillus amyloliquefaciens
<400> 231
   Asn Gly Ile Glu Trp Ala Ile Ala Asn Asn Met Asp Val Ile Asn
   1 5 10 15
<210> 232
   <211> 15
   <212> PRT
   <213> Bacillus lentus
<400> 232
   Thr Gly Ser Gly Val Lys Val Ala Val Leu Asp Thr Gly Ile Ser
   1 5 10 15
<210> 233
   <211> 15
   <212> PRT
   <213> Bacillus lentus
<400> 233
   Ser Ala Glu Leu Tyr Ala Val Lys Val Leu Gly Ala Ser Gly Ser
   1 5 10 15
<210> 234
   <211> 17
   <212> PRT
   <213> Bacillus lentus
<400> 234
   Gly Ser Ile Ser Tyr Pro Ala Arg Tyr Ala Asn Ala Met Ala Val Gly
   1 5 10 15
   Ala
<210> 235
   <211> 15
   <212> PRT
   <213> Bacillus lentus
<400> 235
   Gly Ala Gly Leu Asp Ile Val Ala Pro Gly Val Asn Val Gln Ser
   1 5 10 15
<210> 236
   <211> 272
   <212> PRT
   <213> Artificial Sequence
<220>
   <223> Description of Artificial Sequence: Hybrid of Bacillus lentus and Bacillus amyloliquefaciens
<400> 236
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
                       20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ile Asn Met Ser Leu Gly Gly Ser Gly
           115                 120                 125
   Ser Ala Ala Leu Lys Ala Ala Val Asp Lys Ala Val Ala Ser Gly Val
       130                 135                 140
   Val Val Val Ala Ala Ala Gly Asn Glu Gly Thr Ser Gly Ser Ser Ser
   145                 150                 155                 160
   Thr Val Gly Tyr Pro Gly Lys Tyr Pro Ser Val Ile Ala Val Gly Ala
                   165                 170                 175
   Val Asp Ser Ser Asn Gln Arg Ala Ser Phe Ser Ser Val Gly Pro Glu
               180                 185                 190
   Leu Asp Val Met Ala Pro Gly Val Ser Ile Gln Ser Thr Leu Pro Gly
           195                 200                 205
   Asn Lys Tyr Gly Ala Tyr Asn Gly Thr Ser Met Ala Ser Pro His Val
       210                 215                 220
   Ala Gly Ala Ala Ala Leu Ile Leu Ser Lys His Pro Asn Trp Thr Asn
   225                 230                 235                 240
   Thr Gln Val Arg Ser Ser Leu Glu Asn Thr Thr Thr Lys Leu Gly Asp
                   245                 250                 255
   Ser Phe Tyr Tyr Gly Lys Gly Leu Ile Asn Val Gln Ala Ala Ala Gln
               260                 265                 270

## Description

### BACKGROUND OF THE INVENTION

Proteins used in industrial, pharmaceutical and commercial applications are of increasing prevalence. As a result, the increased exposure due to this prevalence has been responsible for some safety hazards caused by the sensitization of certain persons to those peptides, whereupon subsequent exposure causes extreme allergic reactions which can be injurious and even fatal. For example, proteases are known to cause dangerous hypersensitivity in some individuals. As a result, despite the usefulness of proteases in industry, e.g., in laundry detergents, cosmetics, textile treatment etc., and the extensive research performed in the field to provide improved proteases which have, for example, more effective stain removal under detergency conditions; the use of proteases in industry has been problematic due to their ability to produce a hypersensitive allergenic response in some humans.

Much work has been done to alleviate these problems. Among the strategies explored to reduce immunogenic potential of protease use have been improved production processes which reduce potential contact by controlling and minimizing workplace concentrations of dust particles or aerosol carrying airborne protease, improved granulation processes which reduce the amount of dust or aerosol actually produced from the protease product, and improved recovery processes to reduce the level of potentially allergenic contaminants in the final product. However, efforts to reduce the allergenicity of protease, per se, have been relatively unsuccessful. Alternatively, efforts have been made to mask epitopes in protease which are recognized by immunoglobulin E (IgE) in hypersensitive individuals (PCT Publication No. WO 92/10755) or to enlarge or change the nature of the antigenic determinants by attaching polymers or peptides/proteins to the problematic protease.

When an adaptive immune response occurs in an exaggerated or inappropriate form, the individual experiencing the reaction is said to be hypersensitive. Hypersensitivity reactions are the result of normally beneficial immune responses acting inappropriately and sometimes cause inflammatory reactions and tissue damage. They can be provoked by many antigens; and the cause of a hypersensitivity reaction will vary from one individual to the next. Hypersensitivity does not normally manifest itself upon first contact with the antigen, but usually appears upon subsequent contact. One form of hypersensitivity occurs when an IgE response is directed against innocuous environmental antigens, such as pollen, dust-mites or animal dander. The resulting release of pharmacological mediators by IgE-sensitized mast cells produces an acute inflammatory reaction with symptoms such as asthma or rhinitis.

Nonetheless, a strategy comprising modifying the IgE sites will not generally be successful in preventing the cause of the initial sensitization reaction. Accordingly, such strategies, while perhaps neutralizing or reducing the severity of the subsequent hypersensitivity reaction, will not reduce the number or persons actually sensitized. For example, when a person is known to be hypersensitive to a certain antigen, the general, and only safe, manner of dealing with such a situation is to isolate the hypersensitive person from the antigen as completely as possible. Indeed, any other course of action would be dangerous to the health of the hypersensitive individual. Thus, while reducing the danger of a specific protein for a hypersensitive individual is important, for industrial purposes it would be far more valuable to render a protein incapable of initiating the hypersensitivity reaction in the first place.

T-lymphocytes (T-cells) are key players in the induction and regulation of immune responses and in the execution of immunological effector functions. Specific immunity against infectious agents and tumors is known to be dependent on these cells and they are believed to contribute to the healing of injuries. On the other hand, failure to control these responses can lead to auto aggression. In general, antigen is presented to T-cells in the form of antigen presenting cells which, through a variety of cell surface mechanisms, capture and display antigen or partial antigen in a manner suitable for antigen recognition by the T-cell. Upon recognition of a specific epitope by the receptors on the surface of the T-cells (T-cell receptors), the T-cells begin a series of complex interactions, including proliferation, which result in the production of antibody by B-cells. While T-cells and B-cells are both activated by antigenic epitopes which exist on a given protein or peptide, the actual epitopes recognized by these mononuclear cells are generally not identical. In fact, the epitope which activates a T-cell to initiate the creation of immunologic diversity is quite often not the same epitope which is later recognized by B-cells in the course of the immunologic response. Thus, with respect to hypersensitivity, while the specific antigenic interaction between the T-cell and the antigen is a critical element in the initiation of the immune response to antigenic exposure, the specifics of that interaction, i.e., the epitope recognized, is often not relevant to subsequent development of a full blown allergic reaction.

PCT Publication No. WO 96/40791 discloses a process for producing polyalkylene oxide-polypeptide conjugates with reduced allergenicity using polyalkylene oxide as a starting material.

PCT Publication No. WO 97/30148 discloses a polypeptide conjugate with reduced allergenicity which comprises one polymeric carrier molecule having two or more polypeptide molecules coupled covalently thereto.

PCT Publication No. WO 96/17929 discloses a process for producing polypeptides with reduced allergenicity comprising the step of conjugating from 1 to 30 polymolecules to a parent polypeptide.

PCT Publication No. WO 92/10755 discloses a method of producing protein variants evoking a reduced immunogenic response in animals. In this application, the proteins of interest, a series of proteases and variants thereof, were used to immunize rats. The sera from the rats was then used to measure the reactivity of the polyclonal antibodies already produced and present in the immunized sera to the protein of interest and variants thereof. From these results, it was possible to determine whether the antibodies in the preparation were comparatively more or less reactive with the protein and its variants, thus permitting an analysis of which changes in the protein are likely to neutralize or reduce the ability of the Ig to bind. From these tests on rats, the conclusion was arrived at that changing any of subtilisin 309 residues corresponding to 127, 128, 129, 130, 131, 151, 136, 151, 152, 153, 154, 161, 162, 163, 167, 168, 169, 170, 171, 172, 173, 174, 175, 176, 186, 193, 194, 195, 196, 197, 247, 251, 261 will result in a change in the immunological potential.

PCT Publication No. WO 94/10191 discloses low allergenic proteins comprising oligomeric forms of the parent monomeric protein, wherein the oligomer has substantially retained its activity.

PCT Publication No. WO99/53038 describes methods for identifying T cell epitopes in proteins, methods of reducing allergenicity of proteins, and subtilisin mutants having reduced allergenicity.

### SUMMARY OF THE INVENTION

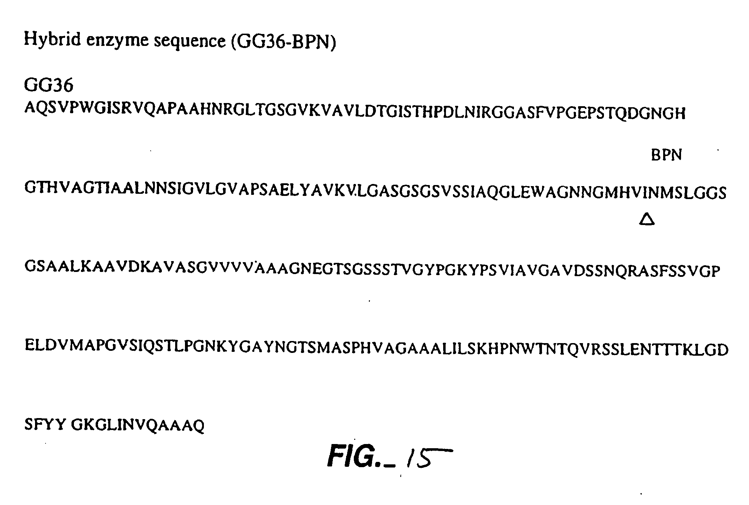

The present invention provides a hybrid protease having the amino acid sequence of Figure 15. The protease is a hybrid between the N-terminal sequence of subtilisin GG36, and the C terminal sequence of subtilisin BPN'.

The invention further provides a nucleic acid encoding said hybrid protease, an expression vector comprising said nucleic acid, and a host cell comprising said expression vector.

In a further aspect, the invention provides a cleaning composition, an animal feed composition, or a composition for treating a textile, comprising, the hybrid protease of claim 1.

### BRIEF DESCRIPTION OF THE DRAWINGS

Figs. 1A, B1, B2 and B3 illustrate the DNA (SEQ ID:NO 1) and amino acid (SEQ ID: NO 2) sequence for *Bacillus amyloliquefaciens* subtilisin (BPN') and a partial restriction map of this gene.

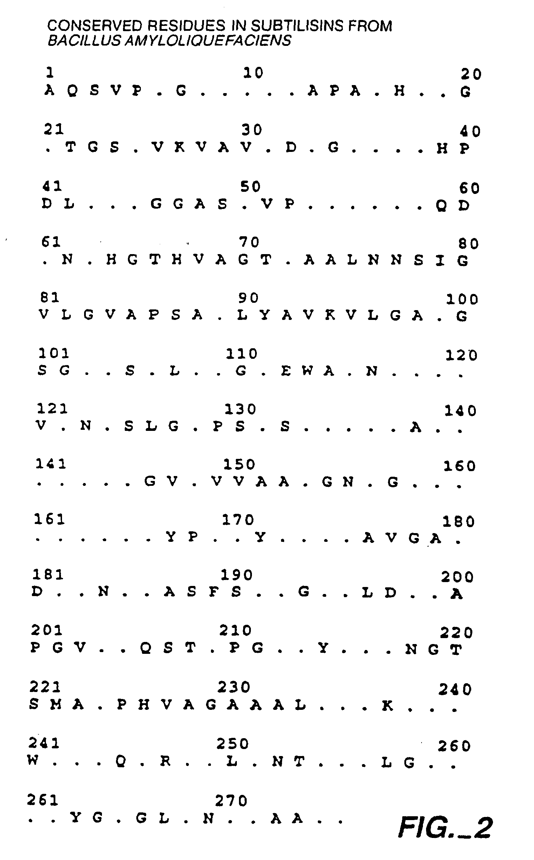

Fig. 2 illustrates the conserved amino acid residues among subtilisins from *Bacillus amyloliquefaciens* (SEQ ID:NO 3) and *Bacillus lentus* (wild-type) (SEO ID:NO 4).

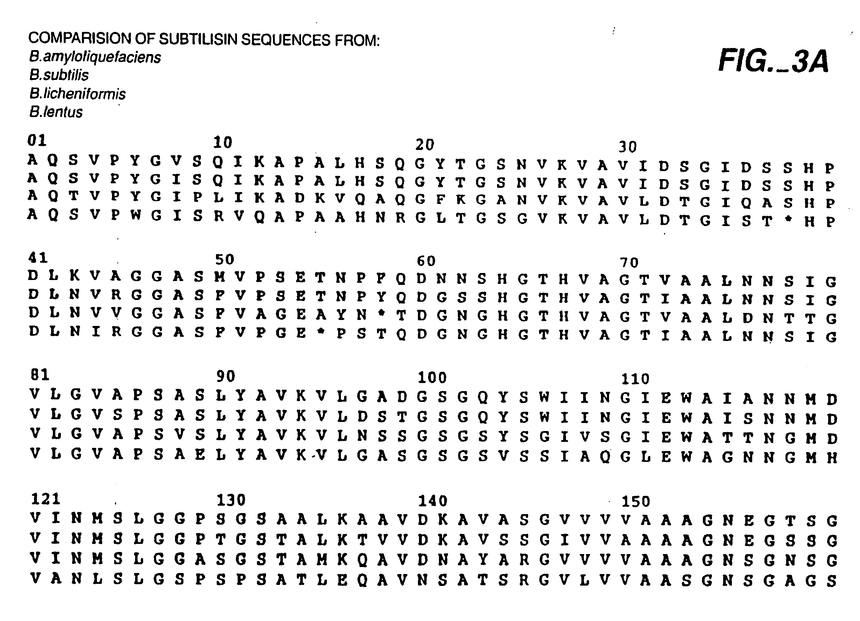

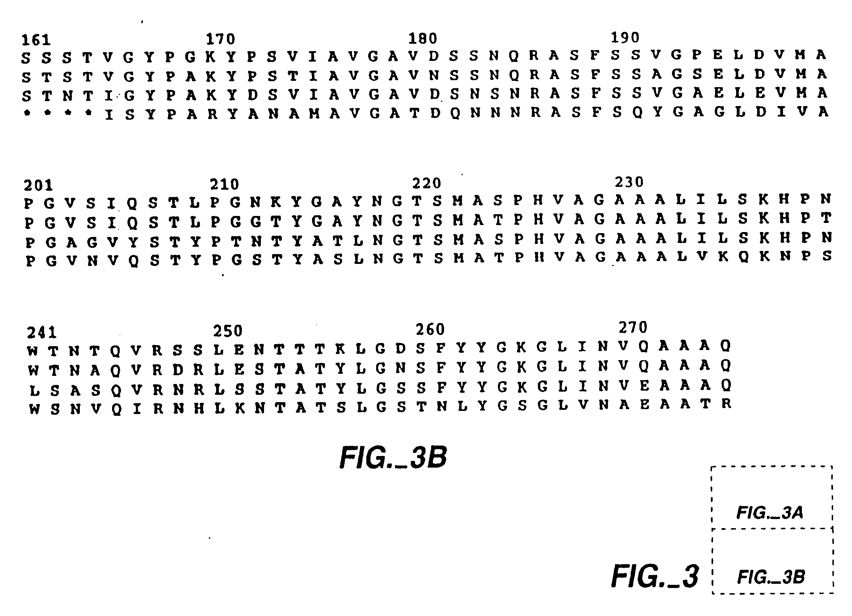

Figs. 3A and 3B illustrate an amino acid sequence alignment of subtilisin type proteases from *Bacillus amyloliquefaciens* (BPN'), *Bacillus subtilis, Bacillus licheniformis* (SEQ ID:NO 5) and *Bacillus lentus.* The symbol * denotes the absence of specific amino acid residues as compared to subtilisin BPN'.
Fig. 4 illustrates the additive T-cell response of 16 peripheral mononuclear blood samples to peptides corresponding to the *Bacillus lentus* protease (GG36). Peptide E05 includes the region comprising residues corresponding to 170-173 in protease from *Bacillus amyloliquefaciens.*
Fig. 5 illustrates the additive T-cell response of 10 peripheral mononuclear blood samples to peptides corresponding to the human subtilisin molecule. Peptides F10, F9, F8 and F7 all contain the amino acid sequence DQMD corresponding to the region comprising residues corresponding to 170-173 in protease from *Bacillus amyloliquefaciens* in the sequence alignment of Fig. 3.
Fig. 6A and 6B/6C illustrate amino acid strings corresponding to peptides derived from the sequence of *Bacillus lentus* protease and a human subtilisin, respectively.
Fig. 7 illustrates the amino acid sequence of human subtilisin (SEQ ID:NO 6).
Fig. 8 illustrates an amino acid sequence alignment of BPN' (*Bacillus amyloliquefaciens*) protease, SAVINASE (*Bacillus lentus*) protease and human subtilisin (S2HSBT).
Fig. 9 illustrates the T-cell response to peptides derived from *Bacillus lentus* protease in a sample taken from an individual known to be hypersensitive to *Bacillus lentus* protease. Peptide E05 represents the region corresponding to 170-173 in protease from *Bacillus amyloliquefaciens.*
Fig. 10 illustrates the T-cell response to various alanine substitutions in the E05 *Bacillus lentus* protease peptide set in a sample taken from an individual known to be hypersensitive to *Bacillus lentus* protease.
Fig. 11 illustrates the T-cell response to various alanine substitutions in the E05 protease peptide (an embodiment of the T-cell epitope designated unmodified sequence) set in a sample taken from an individual known to be hypersensitive to the protease: the sequences for each peptide are also shown.

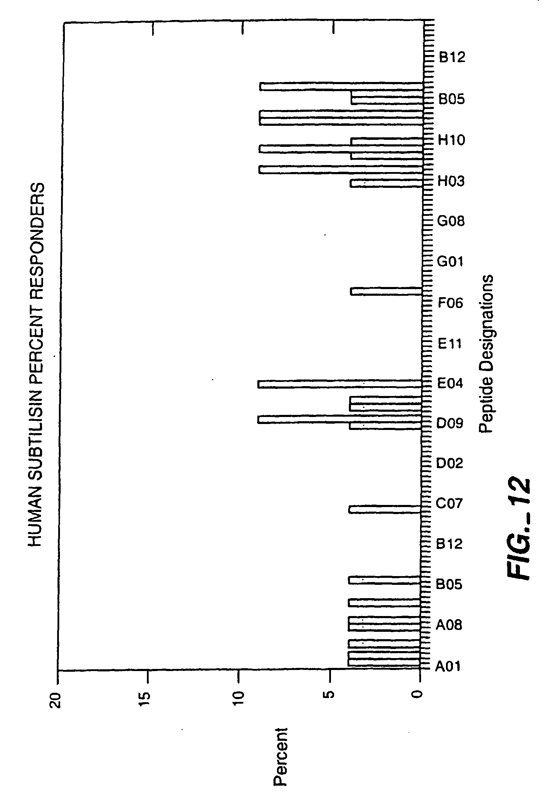

Fig. 12 illustrates the percent responders to the human subtilisin molecule.
Fig. 13 illustrates the T-cell response to peptides derived from BPN' compiled for 22 individuals, wherein the sequences of preferred T-cell epitopes are indicated.
Fig. 14 illustrates the T-cell response to peptides derived from GG36 compiled for 22 individuals, wherein the sequences of embodiments of T-cell epitopes are indicated, GSISYPARYANAMAVGA and GAGLDIVAPGVNVQS being preferred.
Fig. 15 is a hybrid protein, where the N-terminus comprises N-terminal GG36 sequence and the C-terminus comprises C-terminal BPN' sequence, and wherein a comparison of the sequences with those shown in Fig. 8 indicates that the hybrid formed omits preferred T-cell epitopes of each protein.

### DETAILED DESCRIPTION OF THE INVENTION

WO99/53038 describes a method for identifying T-cell epitopes which is illustrated in Examples 1 and 2, below.

In the assay, which identifies epitopes and non-epitopes, differentiated dendritic cells are combined with naïve human CD4+ and/or CDB+ T-cells and with a peptide of interest. More specifically, a T-cell epitope is recognized by (a) obtaining from a single blood source a solution of dendritic cells and a solution of naïve CD4+ and/or CD8⁺ T-cells; (b) promoting differentiation in said solution of dendritic cells; (c) combining said solution of differentiated dendritic cells and said naïve CD4+ and/or CD8+ T-cells with a peptide of interest; (d) measuring the proliferation of T-cells in said step (c).

The peptide of interest to be analyzed is typically derived from a polypeptide of interest. It is possible to identify with precision the location of an epitope which can cause sensitization in an individual or sampling of individuals. A series of peptide oligomers which correspond to all or part of the polypeptide of interest are typically prepared. For example, a peptide library is produced covering the relevant portion or all of the protein.

"Hybrid polypeptides" are proteins engineered from at least two different proteins, which are typically homologs of one another. For example, a hybrid polypeptide might have the N-terminus of a protein and the C-terminus of a homolog of the protein. The two terminal ends can be combined to correspond to the full-length active protein. Typically, the homologs share substantial similarity but do not have identical T-cell epitopes. Therefore, for example, a polypeptide of interest having one or more T-cell epitopes in the C-terminus may have the C-terminus replaced with the C-terminus of a homolog having a less potent T-cell epitope in the C-terminus, less T-cell epitopes, or no T-cell epitope in the C-terminus. Thus, the skilled artisan understands that by being able to identify T-cell epitopes among homologs, variants producing reduced immunogenic responses can be formed. mature form of protein as well as pro- and prepro- forms. The prepro- forms are the preferred construction since this facilitates the expression, secretion and maturation of the protein.

"Prosequence" refers to a sequence of amino acids bound to the N-terminal portion of the mature form of a protein which when removed results in the appearance of the "mature" form of the protein. Many proteolytic enzymes are found in nature as translational proenzyme products and, in the absence of post-translational processing, are expressed in this fashion. A preferred prosequence for producing protein variants such as protease variants is the putative prosequence of *Bacillus amyloliquefaciens* subtilisin, although other prosequences may be used.

A "signal sequence" or "presequence" refers to any sequence of amino acids bound to the N-terminal portion of a protein or to the N-terminal portion of a proprotein which may participate in the secretion of the mature or pro forms of the protein. This definition of signal sequence is a functional one, meant to include all those amino acid sequences encoded by the N-terminal portion of the protein gene which participate in the effectuation of the secretion of protein under native conditions. The present invention utilizes such sequences to effect the secretion of the protein variants as defined herein. One possible signal sequence comprises the first seven amino acid residues of the signal sequence from *Bacillus subtilis* subtilisin fused to the remainder of the signal sequence of the subtilisin from *Bacillus lentus* (ATCC 21536).

A "prepro" form of a protein variant consists of the mature form of the protein having a prosequence operably linked to the amino terminus of the protein and a "pre" or "signal" sequence operably linked to the amino terminus of the prosequence.

"Expression vector" refers to a DNA construct containing a DNA sequence which is operably linked to a suitable control sequence capable of effecting the expression of said DNA in a suitable host. Such control sequences include a promoter to effect transcription, an optional operator sequence to control such transcription, a sequence encoding suitable mRNA ribosome binding sites and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. In the present specification, "plasmid" and "vector" are sometimes used interchangeably as the plasmid is the most commonly used form of vector at present. However, the invention is intended to include such other forms of expression vectors which serve equivalent functions and which are, or become, known in the art.

The "host cells" used in the present invention generally are procaryotic or eucaryotic hosts which preferably have been manipulated by the methods disclosed in US Patent 4,760,025 (RE 34,606) to render them incapable of secreting enzymatically active endoprotease. A preferred host cell for expressing protein is the *Bacillus* strain BG2036 which is deficient in enzymatically active neutral protein and alkaline protease (subtilisin). The construction of strain BG2036 is described in detail in US Patent 5,264,366. Other host cells for expressing protein include *Bacillus subtilis* 1168 (also described in US Patent 4,760,025 (RE 34.606) and US Patent 5,264,366, the disclosure of which are incorporated herein by reference), as well as any suitable *Bacillus* strain such as *B. licheniformis, B. lentus,* etc.

Host cells are transformed or transfected with vectors constructed using recombinant DNA techniques. These techniques can be found in any molecular biology practice guide, for example, Sambrook *et al.* Molecular Cloning - A Laboratory Manual (2nd ed.) Vol. 1-3, Cold Springs Harbor Publishing (1989) ("Sambrook"); and Current Protocols in Molecular Biology, Ausubel *et al.*(eds.), Current Protocols, a joint venture between Greene Publishing Associates, Inc. and John Wiley & Sons, Inc., (1997 Supplement) ("Ausubel"). Such transformed host cells are capable of either replicating vectors encoding the protein variants or expressing the desired protein variant. In the case of vectors which encode the pre- or prepro-form of the protein variant, such variants, when expressed, are typically secreted from the host cell into the host cell medium.

"Operably linked", when describing the relationship between two DNA regions, simply means that they are functionally related to each other. For example, a presequence is operably linked to a peptide if it functions as a signal sequence, participating in the secretion of the mature form of the protein most probably involving cleavage of the signal sequence. A promoter is operably linked to a coding sequence if it controls the transcription of the sequence; a ribosome binding site is operably linked to a coding sequence if it is positioned so as to permit translation.

The genes encoding the naturally-occurring precursor protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

The hybrid protease of the invention is useful in formulating various detergent compositions. A number of known compounds are suitable surfactants useful in compositions comprising the protein mutants of the invention. These include nonionic, anionic, cationic, anionic or zwitterionic detergents, as disclosed in US 4,404,128 to Barry J. Anderson and US 4,261,868 to Jiri Flora, et al. A suitable detergent formulation is that described in Example 7 of US Patent 5,204,015. The art is familiar with the different formulations which can be used as cleaning compositions. In addition to typical cleaning compositions, it is readily understood that the protein variants of the present invention may be used for any purpose that native or wild-type proteins are used. Thus, these variants can be used, for example, in bar or liquid soap applications, dishcare formulations, contact lens cleaning solutions or products, peptide hydrolysis, waste treatment, textile applications, as fusion-cleavage enzymes in protein production, etc. The variants of the present invention may comprise, in addition to decreased allergenicity, enhanced performance in a detergent composition (as compared to the precursor). As used herein, enhanced performance in a detergent is defined as increasing cleaning of certain enzyme sensitive stains such as grass or blood, as determined by usual evaluation after a standard wash cycle.

The hybrid protease of the invention can be formulated into known powdered and liquid detergents having pH between 6.5 and 12.0 at levels of about .01 to about 5% (preferably .1% to .5%) by weight. These detergent cleaning compositions can also include other enzymes such as known proteases, amylases, cellulases, lipases or endoglycosidases, as well as builders and stabilizers.

The addition of proteins, particularly proteases of the invention to conventional cleaning compositions does not create any special use limitation. In other words, any temperature and pH suitable for the detergent is also suitable for the present compositions as long as the pH is within the above range, and the temperature is below the described protein's denaturing temperature. In addition, proteins of the invention can be used in a cleaning composition without detergents, again either alone or in combination with builders and stabilizers.

The hybrid protease of the present invention can be included in animal feed such as part of animal feed additives as described in, for example, US 5,612,055; US 5,314,692; and US 5,147,642.

One aspect of the invention is a composition for the treatment of a textile that includes the hybrid protease of the present invention. The composition can be used to treat for example silk or wool as described in publications such as RD 216,034; EP 134,267; US 4,533.359; and EP 344,259.

In the following Examples, Example 3 illustrates the invention. Example 1 and 2 provide useful background.

### EXAMPLES

### Example 1

### Assay for the Identification of Peptide T-Cell Epitopes Using Naïve Human T-Cells

Fresh human peripheral blood cells were collected from "naïve" humans, *i.e.,* persons not known to be exposed to or sensitized to *Bacillus lentus* protease, for determination of antigenic epitopes in protease from *Bacillus lentus* and human subtilisin. Naive humans is intended to mean that the individual is not known to have been exposed to or developed a reaction to protease in the past. Peripheral mononuclear blood cells (stored at room temperature, no older than 24 hours) were prepared for use as follows: Approximately 30 mls of a solution of buffy coat preparation from one unit of whole blood was brought to 50 ml with Dulbecco's phosphate buffered solution (DPBS) and split into two tubes. The samples were underlaid with 12.5 ml of room temperature lymphoprep density separation media (Nycomed density 1.077 g/ml). The tubes were centrifuged for thirty minutes at 600G. The interface of the two phases was collected, pooled and washed in DPBS. The cell density of the resultant solution was measured by hemocytometer. Viability was measured by trypan blue exclusion.

From the resulting solution, a differentiated dendritic cell culture was prepared from the peripheral blood mononuclear cell sample having a density of 108 cells per 75 ml culture flask in a solution as follows:
(1) 50 ml of serum free AIM V media (Gibco) was supplemented with a 1:100 dilution beta-mercaptoethanol (Gibco). The flasks were laid flat for two hours at 37°C in 5% CO2 to allow adherence of monocytes to the flask wall.
(2) Differentiation of the monocyte cells to dendritic cells was as follows: nonadherent cells were removed and the resultant adherent cells (monocytes) combined with 30 ml of AIM V, 800 units/ml of GM-CSF (Endogen) and 500 units/ml of IL-4 (Endogen); the resulting mixture was cultured for 5 days under conditions at 37°C in 5% CO2. After five days, the cytokine TNFa (Endogen) was added to 0.2 units/ml, and the cytokine IL-1a (Endogen) was added to a final concentration of 50 units/ml and the mixture incubated at 37°C in 5% CO2 for two more days.
(3) On the seventh day, Mitomycin C was added to a concentration of 50 microgram/ml was added to stop growth of the now differentiated dendritic cell culture. The solution was incubated for 60 minutes at 37°C in 5% CO2. Dendritic cells were collected by gently scraping the adherent cells off the bottom of the flask with a cell scraper. Adherent and non-adherent cells were then centrifuged at 600G for 5 minutes, washed in DPBS and counted.
(4) The prepared dendritic cells were placed into a 96 well round bottom array at 2x104/well in 100 microliter total volume of AIM V media.

CD4+ T cells were prepared from frozen aliquots of the peripheral blood cell samples used to prepare the dendritic cells using the human CD4+ Cellect Kit (Biotex) as per the manufacturers instructions with the following modifications: the aliquots were thawed and washed such that approximately 108 cells will be applied per Cellect column; the cells were resuspended in 4 ml DPBS and 1 ml of the Cell reagent from the Cellect Kit, the solution maintained at room temperature for 20 minutes. The resultant solution was centrifuged for five minutes at 600G at room temperature and the pellet resuspended in 2 ml of DPBS and applied to the Cellect columns. The effluent from the columns was collected in 2% human serum in DPBS. The resultant CD4+ cell solution was centrifuged, resuspended in AIMV media and the density counted.

The CD4+ T-cell suspension was resuspended to a count of 2x106/ml in AIM V media to facilitate efficient manipulation of the 96 well plate.

Peptide antigen is prepared from a 1M stock solution in DMSO by dilution in AIM V media at a 1:10 ratio. 10 microliters of the stock solution is placed in each well of the 96 well plate containing the differentiated dendritic cells. 100 microliter of the diluted CD4+ T-cell solution as prepared above is further added to each well. Useful controls include diluted DMSO blanks, and tetanus toxoid positive controls.

The final concentrations in each well, at 210 microliter total volume are as follows:
2x104 CD4+
2x105 dendtritic cells (R:S of 10:1)
5 mM peptide

### Example 2

### Identification of T-Cell Epitopes in Protease from Bacillus lentus and Human subtilisin

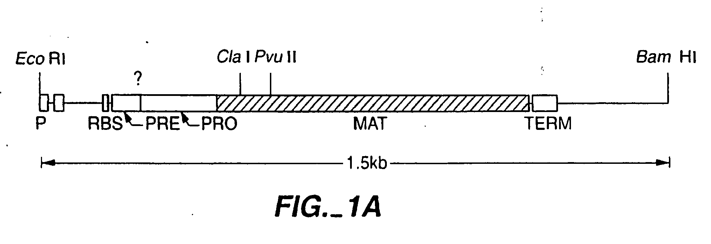

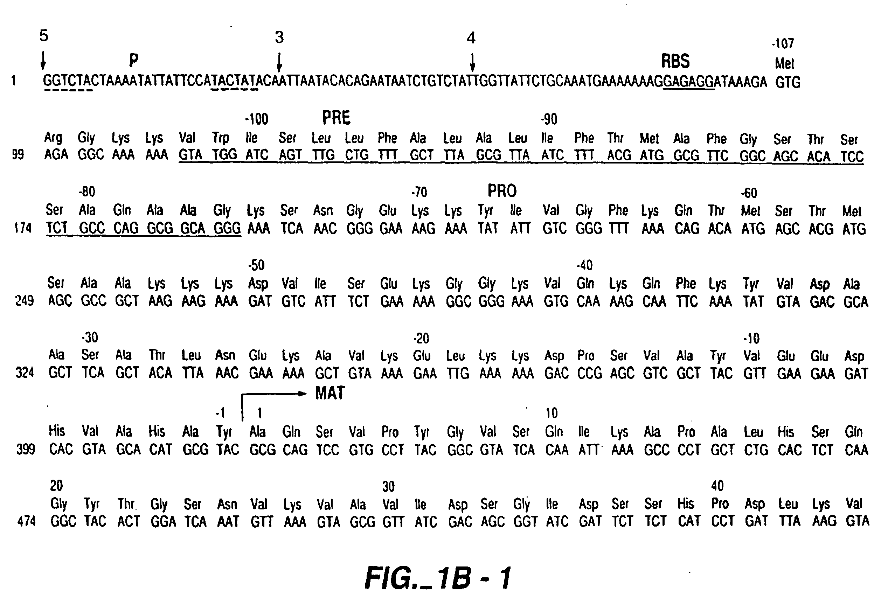

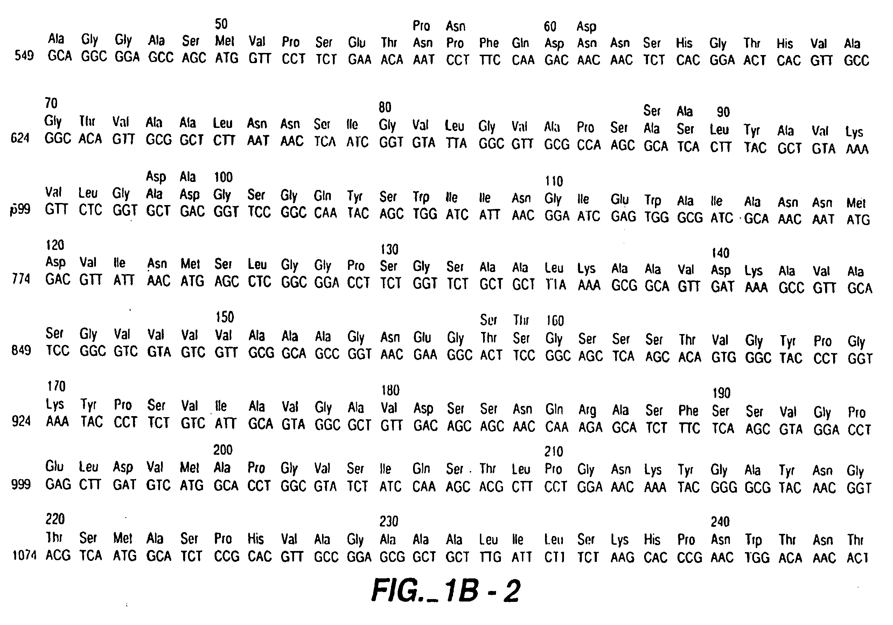

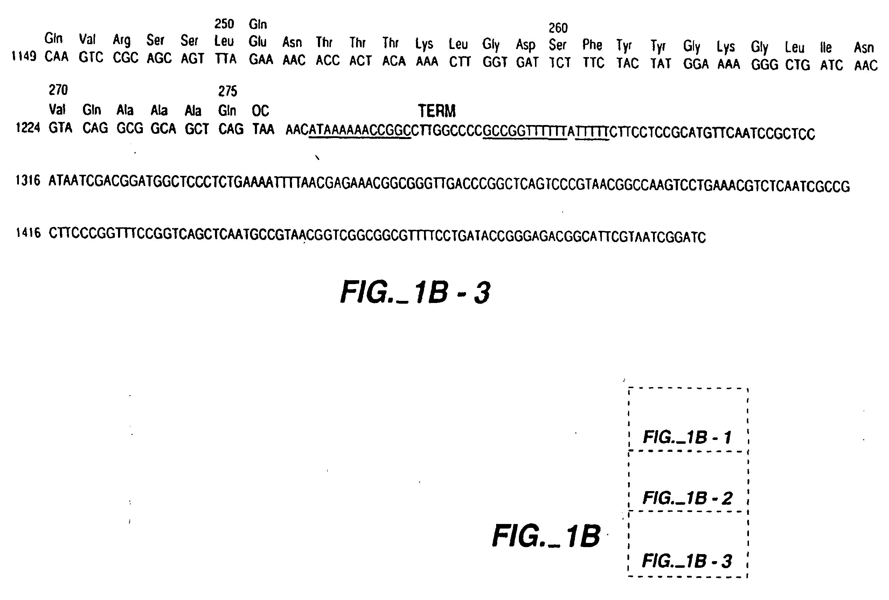

Peptides for use in the assay described in Example 1 were prepared based on the *Bacillus lentus* and human subtilisin amino acid sequence. Peptide antigens were designed as follows. From the full length amino acid sequence of either human subtilisin or *Bacillus lentus* protease provided in Figure 1. 15mers were synthetically prepared, each 15mer overlapping with the previous and the subsequent 15mer except for three residues.

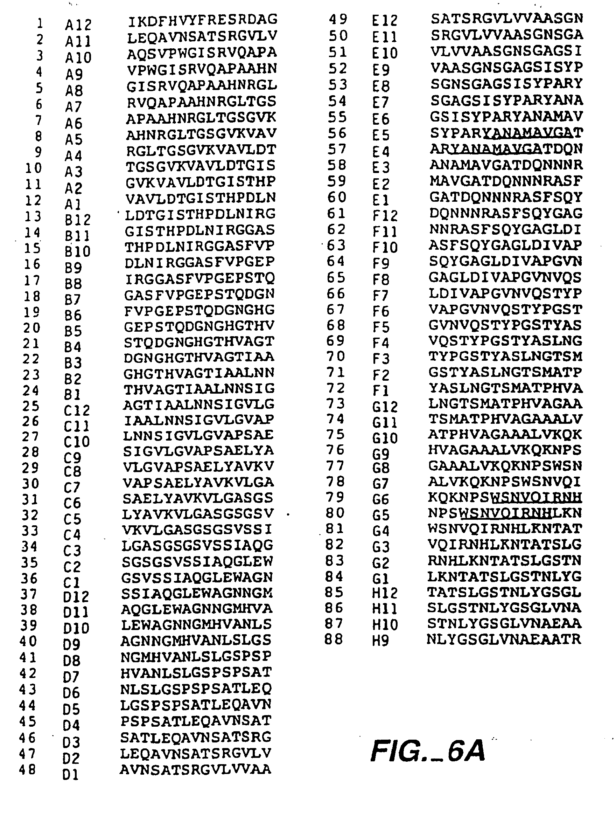

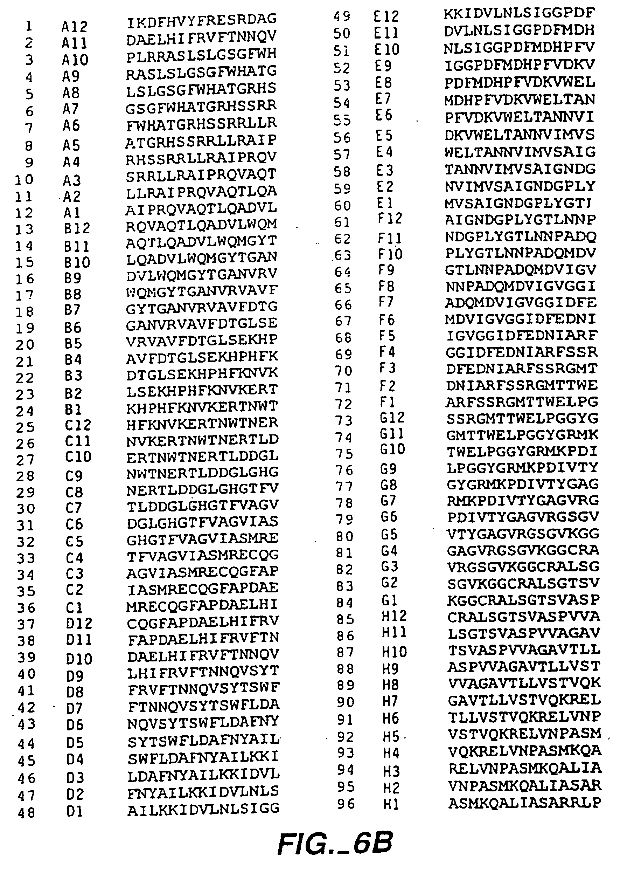

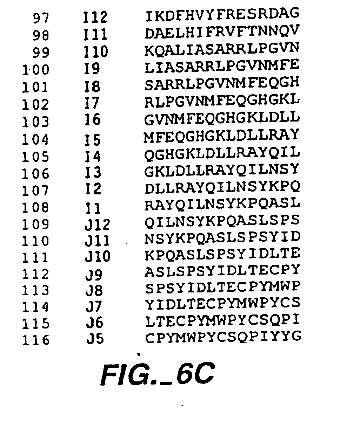

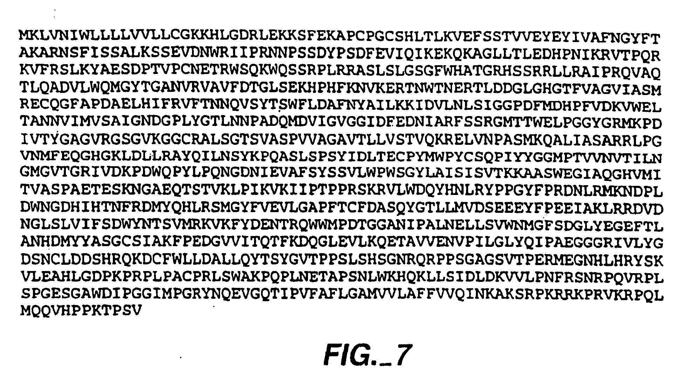

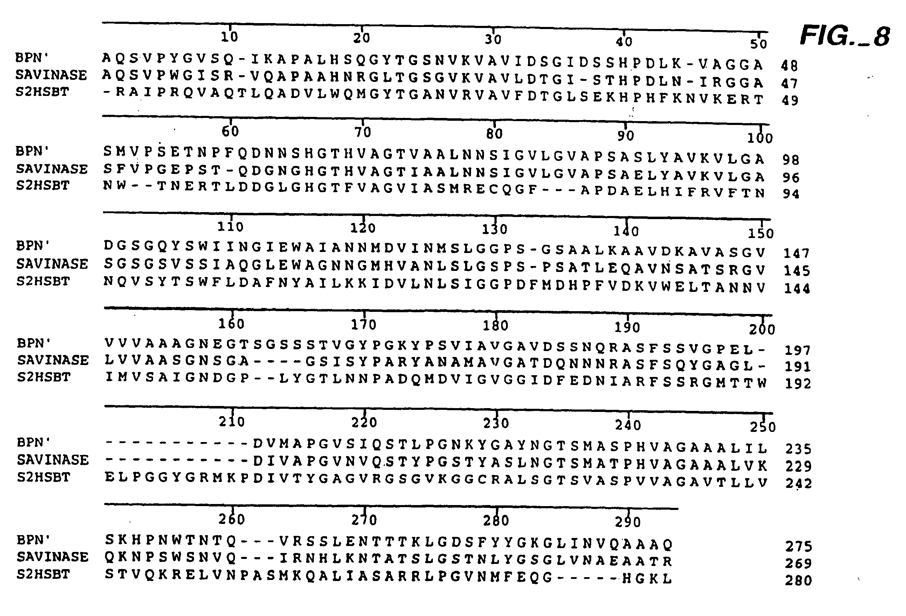

Peptides used correspond to amino acid residue strings in *Bacillus lentus* as provided in Figure 8, and peptides correspond to amino acid residues in human subtilisin as provided in Figure 7. The peptides used corresponding to the proteases is provided in Fig. 6. All tests were performed at least in duplicate. All tests reported displayed robust positive control responses to the antigen tetanus toxoid. Responses were averaged within each experiment, then normalized to the baseline response. A positive event was recorded if the response was at least 3 times the baseline response.

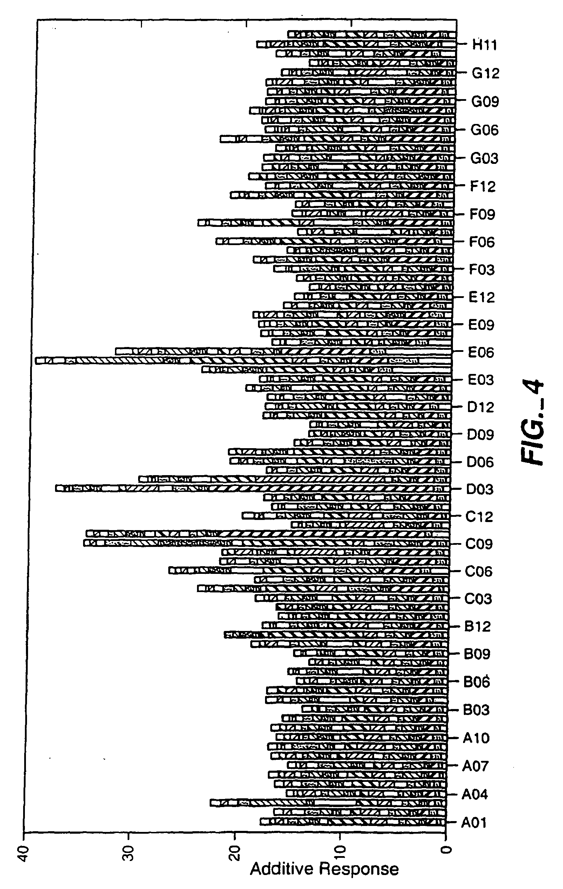

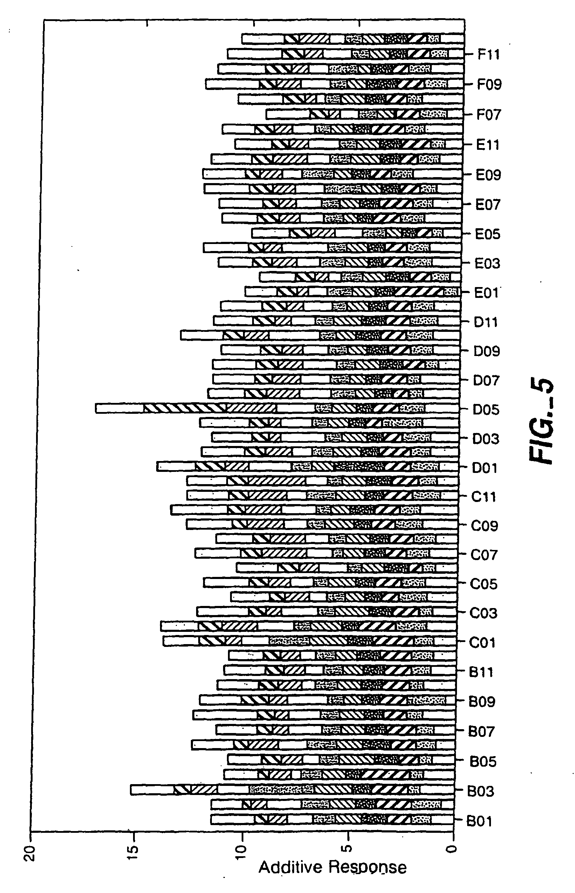

The immunogenic response (*i.e.,* T-cell proliferation) to the prepared peptides from human subtilisin and *Bacillus lentus* was tallied and is provided in Figures 4 and 5, respectively. T-cell proliferation was measured by the incorporated tritium method. The results shown in Figures 4 and 5 as a comparison of the immunogenic additive response in 10 individuals (Figure 4) and 16 individuals (Figure 5) to the various peptides. Response is indicated as the added response wherein 1.0 equals a baseline response for each sample. Thus, in Figure 4, a reading of 10.0 or less is the baseline response and in Figure 5 a reading of 16.0 or less the baseline response. The greater the response, the more potent the T-cell epitope is considered.

As indicated in Figures 4 and 5, the immunogenic response of the naive blood samples from unsensitized individuals showed a marked allergenic response at the peptide fragment from *Bacillus lentus* corresponding to residues 170-173 of *Bacillus amyloliquefaciens* protease. As expected, the corresponding fragment in human subtilisin evokes merely baseline response.

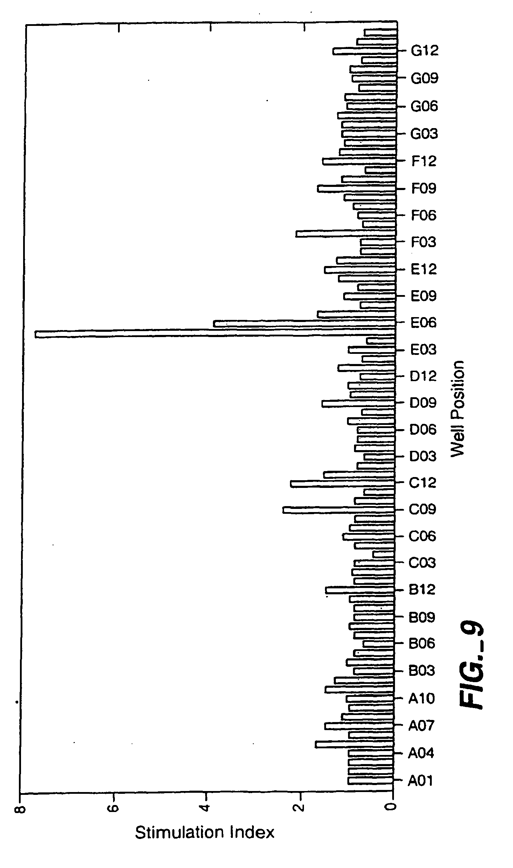

Fig. 9 shows the T-cell response to peptides derived from *Bacillus lentus* protease in a sample taken from an individual known to be hypersensitive to *Bacillus lentus* protease. Peptide E05 represents the region corresponding to 170-173 in protease from *Bacillus amyloliquefaciens.* As shown in Fig. 9, the hypersensitive individual was highly responsive to the T-cell epitope represented by the peptide E05. This result confirms that, by practicing the assay according to the invention, it is possible to predict the major epitopes identified by the T-cells of a hypersensitive individual.

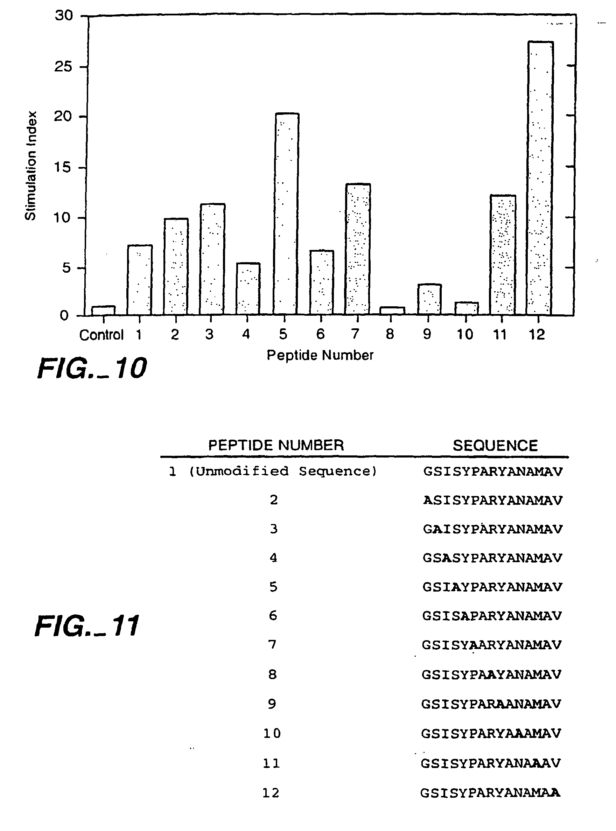

Fig. 10 shows the T-cell response to various alanine substitutions in the E05 peptide derived from *Bacillus lentus* protease in a sample taken from an individual known to be hypersensitive to *Bacillus lentus* protease. Alanine substitutions were used as substitutions for the purpose of determining the role of any specific residue within the epitope. The legend of Figure 10 refers to the position of the peptide in which an alanine was substituted, i.e., in peptide E06 (sequence GSISYPARYANAMAV), G to A=2, S to A=3, I to A= 4, S to A = 5, Y to A = 6, P to A = 7, R to A = 8, Y to A = 9, N to A = 10, M to A = 11 and V to A = 12. As indicated in Figure 10, substitution of either of the residues R170A, Y171A and/or N173A in protease from *Bacillus lentus* results in dramatically reduced response in the hypersensitive individual's blood sample.

From these results, it is apparent that the residues 170, 171 and 173 are largely responsible for the initiation of allergic reaction within the protease from *Bacillus lentus.*

### Example 3

### Identification of T-Cell Epitopes in a Protease Hybrid (GG36-BPN')

After determining the location of a T-cell epitope, a protease hybrid was constructed using established protein engineering techniques. The hybrid was constructed so that a highly allergenic amino acid sequence of the protein was replaced with a corresponding sequence from a less allergenic homolog. In this instance, the first 122 amino acids of the protease were derived from GG36, and the remaining amino acid sequence was derived from BPN'.

The hybrid was first tested from a 100 ppm sample in North American condition in 24 well assay at .5 ppm, superfixed swatches, liquid (Tide KT) at .5 in 24 well assay with 3K swatches, and in the N'N'-dimethyl Casein Assay, 5 g/l DMC in NA detergent, TNBS dectection method.

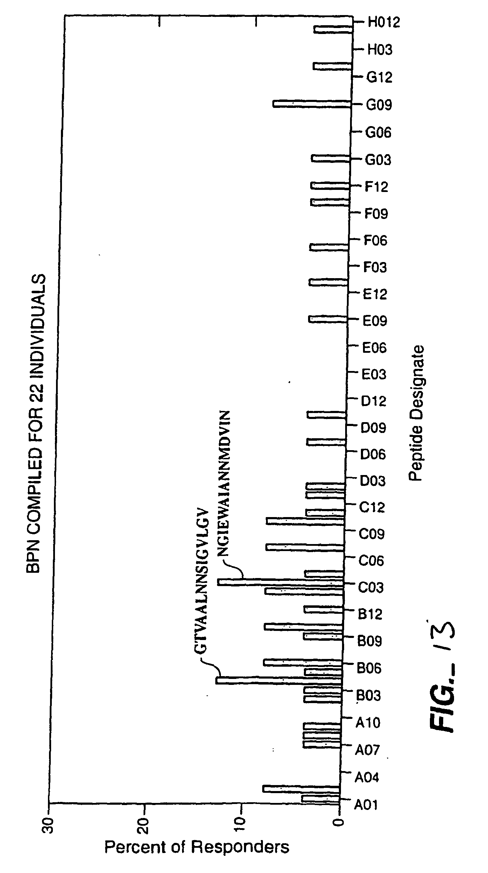

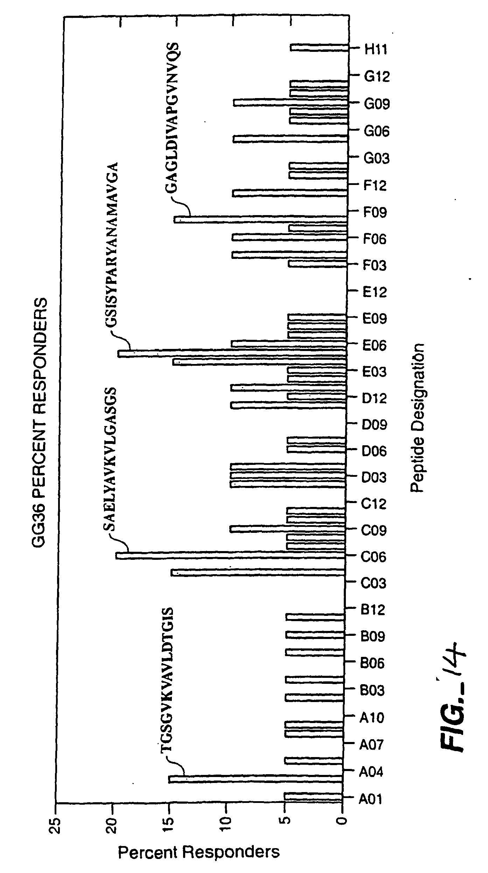

The results are shown in Figures 13, 14 and 15.

## Claims

1. A hybrid protease having the amino acid sequence of Figure 15.

2. A nucleic acid encoding the hybrid protease of claim 1.

3. An expression vector comprising the nucleic acid of claim 2.

4. A host cell comprising the expression vector of claim 3.

5. A cleaning composition, an animal feed composition, or a composition for treating a textile comprising the hybrid protease of claim 1.

## Patentansprüche

1. Hybridprotease mit der Aminosäuresequenz von Figur 15.

2. Nukleinsäure, welche die Hybridprotease von Anspruch 1 codiert.

3. Expressionsvektor, umfassend die Nukleinsäure von Anspruch 2.

4. Wirtszelle, umfassend den Expressionsvektor von Anspruch 3.

5. Reinigungszusammensetzung, Tierfutterzusammensetzung oder eine Zusammensetzung zur Behandlung eines Textils, umfassend die Hybridprotease von Anspruch 1.

## Revendications

1. Protéase hybride ayant 1a séquence d'acides aminés de la séquence 15.

2. Acide nucléique codant pour la protéase hybride de la revendication 1.

3. Vecteur d'expression comprenant l'acide nucléique de la revendication 2.

4. Cellule hôte comprenant le vecteur d'expression de la revendication 3.

5. Composition de nettoyage, composition d'alimentation animale, ou composition destinée à traiter un textile, comprenant la protéase hybride de la revendication 1.
